# EUROPEAN PATENT SPECIFICATION

(11) **EP 3 442 993 B1**
(45) Date of publication and mention of the grant of the patent: **20.01.2021**
(21) Application number: 17718047.8
(22) Date of filing: 12.04.2017
(51) Int. Cl.: C07K 14/005

(54) **METHODS AND KITS FOR THE RAPID DETECTION OF THE ESCHERICHIA COLI O25B-ST131 CLONE**
VERFAHREN UND KITS ZUM SCHNELLEN NACHWEIS DES ESCHERICHIA-COLI-O25B-ST131-KLONS
PROCÉDÉS ET KITS POUR LA DÉTECTION RAPIDE DU CLONE O25B-ST131 ESCHERICHIA COLI

(30) Priority: 13.04.2016 EP 16305433
(43) Date of publication of application: 20.02.2019
(73) Proprietor: INSERM (Institut National de la Santé et de la Recherche Médicale), 75013 Paris (FR); Assistance Publique-Hôpitaux de Paris (APHP), 75004 Paris (FR); Université Paris Diderot - Paris 7, 75013 Paris (FR); Institut Pasteur, 75015 Paris (FR); Université Paris XIII Paris-Nord, 93430 Villetaneuse (FR)
(72) Inventor: RICARD, Jean-Damien, 75870 Paris (FR); DUFOUR, Nicolas, CERGY-PONTOISE 95303 (FR); DENAMUR, Erick, PARIS 75018 (FR); DEBARBIEUX, Laurent, PARIS 75015 (FR); CLERMONT, Olivier, PARIS 75018 (FR)
(74) Representative: Gevers & Orès
(86) International application number: PCT/EP2017/058860
(87) International publication number: WO 2017/178554

(56) References cited:
- WO-A1-2013/045863
- Nicolas Dufour, et al.: "Bacteriophage LM33_P1, a fast-acting weapon against the pandemic ST131-O25b:H4 Escherichia coli clonal complex", J Antimicrob Chemother , 7 July 2016 (2016-07-07), pages 1-9, XP002762144, DOI: 10.1093/jac/dkw253 Retrieved from the Internet: URL:http://jac.oxfordjournals.org/content/ early/2016/07/06/jac.dkw253.full.pdf+html [retrieved on 2016-09-19]
- GALTIER MATTHIEU ET AL: "Bacteriophages to reduce gut carriage of antibiotic resistant uropathogens with low impact on microbiota composition.", ENVIRONMENTAL MICROBIOLOGY, vol. 18, no. 7, July 2016 (2016-07), pages 2237-2245, XP002762145, ISSN: 1462-2920
- F. POUILLOT ET AL: "Efficacy of Bacteriophage Therapy in Experimental Sepsis and Meningitis Caused by a Clone O25b:H4-ST131 Escherichia coli Strain Producing CTX-M-15", ANTIMICROBIAL AGENTS AND CHEMOTHERAPY, vol. 56, no. 7, 1 July 2012 (2012-07-01), pages 3568-3575, XP055285954, US ISSN: 0066-4804, DOI: 10.1128/AAC.06330-11
- V. SZIJARTO ET AL: "Diagnostic Potential of Monoclonal Antibodies Specific to the Unique O-Antigen of Multidrug-Resistant Epidemic Escherichia coli Clone ST131-O25b:H4", CLINICAL AND VACCINE IMMUNOLOGY, vol. 21, no. 7, 30 April 2014 (2014-04-30) , pages 930-939, XP055179667, ISSN: 1556-6811, DOI: 10.1128/CVI.00685-13
- CHIBEU ANDREW ET AL: "Bacteriophages with the Ability to Degrade Uropathogenic Escherichia Coli Biofilms", VIRUSES, vol. 4, no. 4, April 2012 (2012-04), pages 471-487, XP002762148, BASEL ISSN: 1999-4915
- O. CLERMONT ET AL: "Rapid detection of the O25b-ST131 clone of Escherichia coli encompassing the CTX-M-15-producing strains", JOURNAL OF ANTIMICROBIAL CHEMOTHERAPY, vol. 64, no. 2, 1 August 2009 (2009-08-01) , pages 274-277, XP055056612, ISSN: 0305-7453, DOI: 10.1093/jac/dkp194
- M.-H. NICOLAS-CHANOINE ET AL: "Escherichia coli ST131, an Intriguing Clonal Group", CLINICAL MICROBIOLOGY REVIEWS., vol. 27, no. 3, 30 June 2014 (2014-06-30), pages 543-574, XP055286007, US ISSN: 0893-8512, DOI: 10.1128/CMR.00125-13

### SEQUENCE LISTING

<110> Inserm
<120> A BACTERIOPHAGE STRAIN CAPABLE OF PRODUCING A LYTIC INFECTION IN THE ESCHERICHIA COLI ST131-O25B:H4 CLONE
<130> BIO15106 RICARD / MC
<160> 1
<170> PatentIn version 3.5
<210> 1
   <211> 726
   <212> PRT
   <213> Artificial Sequence
<220>
   <223> bacteriophage tail fiber
<400> 1
   Met Ser Thr Ile Thr Gln Phe Pro Ser Gly Asn Thr Gln Tyr Arg Ile
   1               5                   10                  15
   Glu Phe Asp Tyr Leu Ala Arg Thr Phe Val Val Val Thr Leu Val Asn
               20                  25                  30
   Ser Ser Asn Pro Thr Leu Asn Arg Val Leu Glu Val Gly Arg Asp Tyr
           35                  40                  45
   Arg Phe Leu Asn Pro Thr Met Ile Glu Met Leu Ala Asp Gln Ser Gly
       50                  55                  60
   Phe Asp Ile Val Arg Ile His Arg Gln Thr Gly Thr Asp Leu Val Val
   65                  70                  75                  80
   Asp Phe Arg Asn Gly Ser Val Leu Thr Ala Ser Asp Leu Thr Asn Ser
                   85                  90                  95
   Glu Leu Gln Ala Ile His Ile Ala Glu Glu Gly Arg Asp Gln Thr Val
               100                 105                 110
   Asp Leu Ala Lys Glu Tyr Ala Asp Ala Ala Gly Ser Ser Ala Gly Asn
           115                 120                 125
   Ala Lys Asp Ser Glu Asp Glu Ser Arg Arg Ile Ala Ala Ser Ile Lys
       130                 135                 140
   Ala Ala Gly Lys Ile Gly Tyr Ile Thr Arg Arg Ser Phe Glu Lys Gly
   145                 150                 155                 160
   Phe Asn Val Thr Thr Trp Asn Glu Val Leu Leu Trp Glu Glu Asp Gly
                   165                 170                 175
   Asp Tyr Tyr Arg Trp Asp Gly Thr Leu Pro Lys Asn Val Pro Ala Gly
               180                 185                 190
   Ser Thr Pro Glu Ser Ser Gly Gly Ile Gly Leu Ser Ala Trp Val Ser
           195                 200                 205
   Val Gly Asp Ala Ser Leu Arg Ala Asn Leu Ala Asp Ser Asp Gly Ala
       210                 215                 220
   Lys Tyr Ile Gly Ser Gly Glu Arg Thr Leu Leu Glu His Asn Asn Asp
   225                 230                 235                 240
   Val Leu His Ser Lys Asp Phe Pro Thr Leu Gln Ala Ala Ile Asp Ala
                   245                 250                 255
   Ser Leu Gln Lys Asn Asp Leu Leu Val Ser Pro Gly Asn Tyr Thr Glu
               260                 265                 270
   Lys Val Thr Ile Gly Asn Ala Gln Leu Lys Gly Val Gly Gly Ala Thr
           275                 280                 285
   Val Leu Lys Thr Pro Ala Asp Phe Thr Asn Thr Val Gln Val Asn Leu
       290                 295                 300
   Ala Thr Pro His Trp Gln Phe Arg His Ser Gly Gly Phe Ala Ile Asp
   305                 310                 315                 320
   Gly Ser Gly Thr Thr Gly Ala Val Gly Ile Ser Phe Asp Pro Ser Asp
                   325                 330                 335
   Gln Tyr Ser Gly Arg His Asn Phe Ser Asp Val Tyr Ile His Asn Ile
               340                 345                 350
   Asn Lys Ala Ile Gln Lys Pro Ser Gly Asn Ile Gly Asn Thr Trp Arg
           355                 360                 365
   Asn Ile Gly Ile Ser Thr Cys Asp Trp Gly Tyr Tyr Ala Ile Ser Gly
       370                 375                 380
   Ser Glu Met His Cys Gly Ala Asp Thr Leu Tyr Asn Ile His Phe Asp
   385                 390                 395                 400
   Gly Ile Ser Thr Tyr Ala Val Tyr Leu Asp Gly Thr Val Asp Asn Gly
                   405                 410                 415
   Gly Gly Gly Ala Trp Trp Leu Lys Asp Ser Ile Ile Glu Ala Ser Gly
   420 425 430
   Gly Gly Gly Ile Tyr Leu Lys Ser Lys Ser Gly Asp Cys Pro Thr Ser
   435 440 445
   Pro Cys Gly Val Ser Asn Ile Trp Met Glu Ala Ile Ala Thr Ser Pro
   450 455 460
   Ala Val Gln Val Asp Gly Val Ala Gln Lys Pro Arg Val Leu Lys Leu
   465 470 475 480
   Val Asp Thr Ala Ile Phe Phe Ala Glu Tyr Ser Tyr Leu Asn Asn Ile
   485 490 495
   Glu Leu Ser Asn Ser Asn Leu Val Thr Tyr Gly Cys Arg Phe Asp Asn
   500 505 510
   Ala Asp Gly Asn Gln Asp Ile Val Val Asp Ala Gln Ser Thr Ile Val
   515 520 525
   Ala His Asp Val Tyr Leu Asn Gly Ser Ser Gly Lys Asp Val Ile Val
   530 535 540
   Glu Ser Val Ala Ser Gln Ser Ala Thr Ile Ala Ala Thr Asn Leu Ser
   545 550 555 560
   Leu Arg Gly Asn Leu Thr Arg Gly Arg Val Phe Asn Thr Pro Thr Gly
   565 570 575
   Asn Lys Leu Met Ala Ile Thr Phe Asp Ser Gly Ser His Asn Phe Ser
   580 585 590
   Gly Ser Gly Thr Val Asn Gly Ser Thr Val Ser Asp Gly Leu His Ala
   595 600 605
   Ala Thr Cys Thr Glu Phe Ser Phe Pro Gly Ala Gly Leu Tyr Glu Met
   610 615 620
   Val Ala Thr Arg Thr Thr Ile Thr Ser Gly Arg Trp Tyr Val Trp Gly
   625 630 635 640
   Val Asn Ser Arg Leu Gln Ser Gly Ser Ala Asp Ile Ser Ile Thr Ser
   645 650 655
   Gly Ile Thr Met Gly Ser Val Tyr Thr Lys Pro Gly Glu Trp Ile Ser
   660 665 670
   Thr Phe Gly Val Gly Lys Ala Ser Thr Thr Gly Thr Val Ala Leu Tyr
           675                 680                 685
   Val Ser Thr Gly Gly Gly Ser Gly Ala Thr Val Arg Phe Ser Asp Phe
       690                 695                 700
   Phe Ile Ala Glu Phe Thr Thr Gln Ala Gln Ala Leu Ala Phe Ala Asn
   705                 710                 715                 720
   Ser Arg Met Ser Leu Ser
                   725

## Description

### FIELD OF THE INVENTION:

The present invention relates to methods and kits for the rapid detection of the *Escherichia coli* O25b-ST131 clone.

### BACKGROUND OF THE INVENTION:

*Escherichia coli* O25b-ST131 clone is a worldwide pandemic clone, causing predominantly community-onset antimicrobial-resistant infection. For example, a high prevalence of the clone (∼30%-60%) has been identified amongst fluoroquinolone-resistant *E. coli.* In addition, it potentially harbours a variety of β-lactamase genes; most often, these include CTX-M family β-lactamases, and, less frequently, TEM, SHV and CMY β-lactamases. A broad distribution has been demonstrated amongst antimicrobial-resistant E. coli from human infection in Europe (particularly the UK), North America, Canada, Japan and Korea. The clinical spectrum of disease described is similar to that for other *E. coli,* with urinary tract infection predominant. Description ranges from uncomplicated cystitis to severe infection complicated by bacteraemia, renal abscess and emphysematous pyelonephritis. Other sites of infection have included the respiratory tract, ascitic fluid, intra-abdominal abscess, bones/joints and bacteraemia without a clinically apparent focus. *E coli* O25b-ST131 clone has also been reported as a prominent cause of *E. coli* neonatal sepsis. The clone thus constitutes a major public health concern and there is an unmet need for the development of new method for detecting it all the more than phenotypic detection of the ST131 clone is not possible and DNA-based techniques, including MLST and PCR, remains time consuming Szijarto V et al. (2014): "Diagnostic Potential of Monoclonal Antibodies Specific to the Unique O-Antigen of Multidrug-Resistant Epidemic Escherichia coli Clone ST131-025b:H4", Clinical and Vaccine Immunology, Vol. 21, No. 7, pp. 939-939, doi:10.1128/CVI.00685-13, describe antibodies as diagnostic tools for E.coli 025b:ST131 infections.

Clermont O et al. (2009): "Rapid detection of the 025b-ST131 clone of Escherichia coli encompassing the CTX-M-15-producing strains", Journal of Antimicrobial Chemotherapy, Vol. 64, No.2, pp. 274-277,doi:10.1093/jac/dkp194, describe a PCR-based detection scheme for identification of E.coli strain 025b:ST131.

### SUMMARY OF THE INVENTION:

The present invention relates to methods and kits for the rapid detection of the *Escherichia coli* O25b-ST131 clone. In particular, the present invention is defined by the claims.

### DETAILED DESCRIPTION OF THE INVENTION:

The inventors have isolated a *podoviridae* bacteriophage (LM33_P1) infecting the *E. coli* strain LM33 isolated from ventilator associated pneumonia and which belongs to clone STI31-025b. Out of 70 O25b strains tested, LM33_P1 was able to infect 73% of them. By testing different strains of *E coli* belonging to 129 others various distinct serotypes (including twelve O25a) the inventors found that bacteriophage LM33_P1 is able to infect exclusively O25b strains (none of non-O25b strains could be infected by LM33_P1). The inventors have determined that the specificity displayed by bacteriophage LM33_P1 to infect only O25b serotype strains is based on a very specific polypeptide (Gp17) used by LM33_P1 to attach the bacterial cell via LPS molecule.

Accordingly a first object of the present invention relates to a polypeptide comprising an amino acid sequence having at least 80% of identity with the amino acid sequence set forth in SEQ ID NO:1.

According to the invention a first amino acid sequence having at least 80% of identity with a second amino acid sequence means that the first sequence has 70; 71; 72; 73; 74; 75; 76; 77; 78; 79; 80; 81; 82; 83; 84; 85; 86; 87; 88; 89; 90; 91; 92; 93; 94; 95; 96; 97; 98; 99 or 100% of identity with the second amino acid sequence. Sequence identity is frequently measured in terms of percentage identity (or similarity or homology); the higher the percentage, the more similar are the two sequences. Methods of alignment of sequences for comparison are well known in the art. Various programs and alignment algorithms are described in: Smith and Waterman, Adv. Appl. Math., 2:482, 1981; Needleman and Wunsch, J. Mol. Biol., 48:443, 1970; Pearson and Lipman, Proc. Natl. Acad. Sci. U.S.A., 85:2444, 1988; Higgins and Sharp, Gene, 73:237-244, 1988; Higgins and Sharp, CABIOS, 5:151-153, 1989; Corpet et al. Nuc. Acids Res., 16:10881-10890, 1988; Huang et al., Comp. Appls Biosci., 8:155-165, 1992; and Pearson et al., Meth. Mol. Biol., 24:307-31, 1994). Altschul et al., Nat. Genet., 6:119-129, 1994, presents a detailed consideration of sequence alignment methods and homology calculations. By way of example, the alignment tools ALIGN (Myers and Miller, CABIOS 4:11-17, 1989) or LFASTA (Pearson and Lipman, 1988) may be used to perform sequence comparisons (Internet Program® 1996, W. R. Pearson and the University of Virginia, fasta20u63 version 2.0u63, release date December 1996). ALIGN compares entire sequences against one another, while LFASTA compares regions of local similarity. These alignment tools and their respective tutorials are available on the Internet at the NCSA Website, for instance. Alternatively, for comparisons of amino acid sequences of greater than about 30 amino acids, the Blast 2 sequences function can be employed using the default BLOSUM62 matrix set to default parameters, (gap existence cost of 11, and a per residue gap cost of 1). When aligning short peptides (fewer than around 30 amino acids), the alignment should be performed using the Blast 2 sequences function, employing the PAM30 matrix set to default parameters (open gap 9, extension gap 1 penalties). The BLAST sequence comparison system is available, for instance, from the NCBI web site; see also Altschul et al., J. Mol. Biol., 215:403-410, 1990; Gish. & States, Nature Genet., 3:266-272, 1993; Madden et al. Meth. Enzymol., 266:131-141, 1996; Altschul et al., Nucleic Acids Res., 25:3389-3402, 1997; and Zhang & Madden, Genome Res., 7:649-656, 1997.

In some embodiments, the polypeptide consists of the amino acid sequence set forth in SEQ ID NO:1.

In particular the polypeptide of the present invention is a functional conservative variant of the polypeptide consisting of SEQ IID NO: 1. As used herein the term "function-conservative variant" are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Accordingly, a "function-conservative variant" also includes a polypeptide which has the same or substantially similar properties or functions as the native or parent protein to which it is compared (i.e. binding to the LPS molecules of the *E coli* O25b-ST131 clone.). Functional properties of the polypeptide of the present invention could typically be assessed in any functional assay as described in EXAMPLE.

In some embodiments, the polypeptide of the present invention is fused to at least one heterologous polypeptide (i.e. a polypeptide which is not derived from SEQ ID NO:1) to create a fusion protein. The term "fusion protein" refers to the polypeptide according to the invention that is fused directly or via a spacer to at least one heterologous polypeptide. In some embodiments, the fusion protein comprises the polypeptide according to the invention that is fused either directly or via a spacer at its C-terminal end to the N-terminal end of the heterologous polypeptide, or at its N-terminal end to the C-terminal end of the heterologous polypeptide. As used herein, the term "directly" means that the (first or last) amino acid at the terminal end (N or C-terminal end) of the polypeptide is fused to the (first or last) amino acid at the terminal end (N or C-terminal end) of the heterologous polypeptide. In other words, in this embodiment, the last amino acid of the C-terminal end of said polypeptide is directly linked by a covalent bond to the first amino acid of the N-terminal end of said heterologous polypeptide, or the first amino acid of the N-terminal end of said polypeptide is directly linked by a covalent bond to the last amino acid of the C-terminal end of said heterologous polypeptide. As used herein, the term "spacer" refers to a sequence of at least one amino acid that links the polypeptide of the present invention to the heterologous polypeptide. Such a spacer may be useful to prevent steric hindrances. The linker is typically a spacer peptide and will, according to the invention, be selected so as to allow binding of the polypeptide to the heterologous polypeptide. Suitable spacers will be clear to the skilled person based on the disclosure herein, optionally after some limited degree of routine experimentation. Suitable spacers are described herein and may - for example and without limitation - comprise an amino acid sequence, which amino acid sequence preferably has a length of 2 or more amino acids. Typically, the spacer has 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids. However, the upper limit is not critical but is chosen for reasons of convenience regarding e.g. production of such fusion proteins. One useful group of spacer sequences are spacers derived from the hinge region of heavy chain antibodies as described in WO 96/34103 and WO 94/04678. Other examples are poly-alanine spacer sequences such as Ala-Ala-Ala. Further preferred examples of spacer sequences are Gly/Ser spacers of different length including (gly4ser)3 , (gly4ser)4, (gly4ser), (gly3ser), gly3, and (gly3ser2)3.

In some embodiments, the polypeptide of the present invention is fused to an immunoglobulin domain. For example the fusion protein of the present invention may comprise a polypeptide of the present invention that is fused to an Fc portion (such as a human Fc). Said Fc portion may be useful for increasing the production of the polypeptide of the present invention or for mobilizing the polypeptide of the present invention to a solid support. In some embodiments, the polypeptide of the present invention is fused to one or more (typically human) CH1, and/or CH2 and/or CH3 domains, optionally via a spacer sequence. For instance, the polypeptide of the present invention fused to a suitable CH1 domain could for example be used - together with suitable light chains - to generate antibody fragments/structures analogous to conventional Fab fragments or F(ab')2 fragments, but in which one or (in case of an F(ab')2 fragment) one or both of the conventional VH domains have been replaced by a polypeptide of the present invention. In some embodiments, one or more single domain antibodies of the invention may be fused to one or more constant domains (for example, 2 or 3 constant domains that can be used as part of/to form an Fc portion), to an Fc portion and/or to one or more antibody parts, fragments or domains that confer one or more effector functions and/or may confer the ability to bind to one or more Fc receptors. For example, for this purpose, and without being limited thereto, the one or more further amino acid sequences may comprise one or more CH2 and/or CH3 domains of an antibody, such as from a heavy chain antibody and more typically from a conventional human chain antibody; and/or may form and Fc region, for example from IgG (e.g. from IgG1, IgG2, IgG3 or IgG4), from IgE or from another human Ig such as IgA, IgD or IgM.

In some embodiments, the heterologous polypeptide is a fluorescent polypeptide. Suitable fluorescent polypeptides include, but are not limited to, a green fluorescent protein (GFP), including, but not limited to, a "humanized" version of a GFP, e.g., wherein codons of the naturally-occurring nucleotide sequence are changed to more closely match human codon bias; a GFP derived from *Aequoria victoria* or a derivative thereof, e.g., a "humanized" derivative such as Enhanced GFP, which are available commercially, e.g., from Clontech, Inc.; a GFP from another species such as Renilla reniformis, Renilla mulleri, or Ptilosarcus guernyi, as described in, e.g., WO 99/49019 and Peelle et al. (2001) J. Protein Chem. 20:507-519; "humanized" recombinant GFP (hrGFP) (Stratagene); any of a variety of fluorescent and colored proteins from Anthozoan species, as described in, e.g., Matz et al. (1999) Nature Biotechnol. 17:969-973; and the like.

In some embodiments, the heterologous polypeptide is an enzyme. Typically, said enzyme may be selected from the group consisting of β-galactosidase, alkaline phosphatase, luciferase, and horse radish peroxidase). Where the heterologous polypeptide is an enzyme that yields a detectable product, the product can be detected using an appropriate means, e.g., β-galactosidase can, depending on the substrate, yield colored product, which is detected spectrophotometrically, or a fluorescent product; luciferase can yield a luminescent product detectable with a luminometer; etc.

In some embodiments, the heterologous polypeptide is a polypeptide that facilitates purification or isolation of the fusion protein, e.g., metal ion binding polypeptides such as 6H is tags (e.g., acetylated Tat/6His), or glutathione-S-transferase.

The polypeptide of the present invention (fused or not to the heterologous polypeptide) is produced by any technique known in the art, such as, without limitation, any chemical, biological, genetic or enzymatic technique, either alone or in combination. For example, knowing the amino acid sequence of the desired sequence, one skilled in the art can readily produce said polypeptide (fused or not to the heterologous polypeptide), by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions. Alternatively, the polypeptide of the present invention (fused or not to the heterologous polypeptide) can be synthesized by recombinant DNA techniques well-known in the art. For example, the polypeptide of the present invention (fused or not to the heterologous polypeptide) can be obtained as DNA expression products after incorporation of DNA sequences encoding the polypeptide (fused or not to the heterologous polypeptide) into expression vectors and introduction of such vectors into suitable eukaryotic or prokaryotic hosts that will express the desired polypeptide, from which they can be later isolated using well-known techniques. A variety of expression vector/host systems may be utilized to contain and express the polypeptide of the present invention (fused or not to the heterologous polypeptide). These include but are not limited to microorganisms such as bacteria transformed with recombinant bacteriophage, plasmid or cosmid DNA expression vectors; yeast transformed with yeast expression vectors (Giga-Hama et al., 1999); insect cell systems infected with virus expression vectors (e.g., baculovirus, see Ghosh et al., 2002); plant cell systems transfected with virus expression vectors (e.g., cauliflower mosaic virus, CaMV; tobacco mosaic virus, TMV) or transformed with bacterial expression vectors (e.g., Ti or pBR322 plasmid; see e.g., Babe et al., 2000); or animal cell systems. Those of skill in the art are aware of various techniques for optimizing expression of proteins, see e.g., Kaufman, 2000; Colosimo et al., 2000. In the recombinant production of the polypeptide of the present invention (fused or not to the heterologous polypeptide), it would be necessary to employ vectors comprising polynucleotide molecules for encoding said polypeptide. Methods of preparing such vectors as well as producing host cells transformed with such vectors are well known to those skilled in the art. The polynucleotide molecules used in such an endeavour may be joined to a vector, which generally includes a selectable marker and an origin of replication, for propagation in a host. These elements of the expression constructs are well known to those of skill in the art. Generally, the expression vectors include DNA encoding the given protein being operably linked to suitable transcriptional or translational regulatory sequences, such as those derived from a mammalian, microbial, viral, or insect genes. Examples of regulatory sequences include transcriptional promoters, operators, or enhancers, mRNA ribosomal binding sites, and appropriate sequences which control transcription and translation. The terms "expression vector," "expression construct" or "expression cassette" are used interchangeably throughout this specification and are meant to include any type of genetic construct containing a nucleic acid coding for a gene product in which part or all of the nucleic acid encoding sequence is capable of being transcribed. The choice of a suitable expression vector for expression of polypeptide of the present invention will of course depend upon the specific host cell to be used, and is within the skill of the ordinary artisan. Typically, the nucleotide sequences are operably linked when the regulatory sequence functionally relates to the DNA encoding the protein of interest (e.g., a polypeptide). Thus, a promoter nucleotide sequence is operably linked to a given DNA sequence if the promoter nucleotide sequence directs the transcription of the sequence. They may then, if necessary, be purified by conventional procedures, known in themselves to those skilled in the art, for example by fractional precipitation, in particular ammonium sulphate precipitation, electrophoresis, gel filtration, affinity chromatography, etc. In particular, conventional methods for preparing and purifying recombinant proteins may be used for producing the proteins in accordance with the invention. Typically, the nucleic acid molecule or the vector of the present invention include "control sequences"', which refers collectively to promoter sequences, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control sequences need always be present so long as the selected coding sequence is capable of being replicated, transcribed and translated in an appropriate host cell. Another nucleic acid sequence, is a "promoter" sequence, which is used herein in its ordinary sense to refer to a nucleotide region comprising a DNA regulatory sequence, wherein the regulatory sequence is derived from a gene which is capable of binding RNA polymerase and initiating transcription of a downstream (3'-direction) coding sequence. Transcription promoters can include "inducible promoters" (where expression of a polynucleotide sequence operably linked to the promoter is induced by an analyte, cofactor, regulatory protein, etc.), "repressible promoters" (where expression of a polynucleotide sequence operably linked to the promoter is induced by an analyte, cofactor, regulatory protein, etc.), and "constitutive promoters".

A further object of the present invention relates to a nucleic acid molecule which encodes for a polypeptide of the present invention (fused or not to the heterologous polypeptide).

As used herein, the term "nucleic acid molecule" has its general meaning in the art and refers to a DNA or RNA molecule. However, the term captures sequences that include any of the known base analogues of DNA and RNA such as, but not limited to 4-acetylcytosine, 8-hydroxy-N6-methyladenosine, aziridinylcytosine, pseudoisocytosine, 5-(carboxyhydroxylmethyl) uracil, 5-fiuorouracil, 5-bromouracil, 5-carboxymethylaminomethyl-2-thiouracil, 5-carboxymethyl-aminomethyluracil, dihydrouracil, inosine, N6-isopentenyladenine, 1 -methyladenine, 1 -methylpseudouracil, 1-methylguanine, 1- methylinosine, 2,2-dimethylguanine, 2-methyladenine, 2-methylguanine, 3-methylcytosine, 5- methylcytosine, N6-methyladenine, 7-methylguanine, 5-methylaminomethyluracil, 5-methoxyamino-methyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, -uracil-5-oxyacetic acid methylester, uracil-5-oxyacetic acid, pseudouracil, queosine, 2-thiocytosine, and 2,6-diaminopurine.

In some embodiments, the nucleic acid molecule of the present invention is thus included in a suitable vector, such as a plasmid, cosmid, episome, artificial chromosome, phage or a viral vector. So, a further object of the invention relates to a vector comprising a nucleic acid encoding for a polypeptide of the present invention (fused or not to the heterologous polypeptide).

A further object of the present invention relates to a host cell transformed with the nucleic acid molecule of the present invention. The term "transformation" means the introduction of a "foreign" (i.e. extrinsic or extracellular) gene, DNA or RNA sequence to a host cell, so that the host cell will express the introduced gene or sequence to produce a desired substance, i.e. the polypeptide encoded by the introduced gene or sequence. A host cell that receives and expresses introduced DNA or RNA has been "transformed". For instance, as disclosed above, for expressing and producing the polypeptide of the present invention, prokaryotic cells and, in particular *E. coli* cells, will be chosen. Actually, according to the invention, it is not mandatory to produce the polypeptides of the present invention in a eukaryotic context that will favour post-translational modifications (e.g. glycosylation). Typically, the host cell may be suitable for producing the polypeptide of the present invention (fused or not to the heterologous polypeptide) as described above.

Accordingly, in some embodiments, the polypeptide of the present invention (fused or not to the heterologous polypeptide) is conjugated with a detectable label. Suitable detectable labels include, for example, a radioisotope, a fluorescent label, a chemiluminescent label, an enzyme label, a bio luminescent label or colloidal gold. Methods of making and detecting such detectably-labeled immunoconjugates are well-known to those of ordinary skill in the art, and are described in more detail below. For instance, the detectable label can be a radioisotope that is detected by autoradiography. Isotopes that are particularly useful for the purpose of the present invention are ³H, ¹²⁵I, ¹³¹I, ³⁵S and ¹⁴C. The polypeptide of the present invention (fused or not to the heterologous polypeptide) can also be labeled with a fluorescent compound. The presence of a fluorescently-labeled polypeptide of the present invention is determined by exposing the immuno conjugate to light of the proper wavelength and detecting the resultant fluorescence. Fluorescent labeling compounds include fluorescein isothiocyanate, rhodamine, phycoerytherin, phycocyanin, allophycocyanin, o-phthaldehyde and fluorescamine and Alexa Fluor dyes. Alternatively, the polypeptide of the present invention can be detectably labeled by coupling said polypeptide to a chemiluminescent compound. The presence of the chemiluminescent-tagged immuno conjugate is determined by detecting the presence of luminescence that arises during the course of a chemical reaction. Examples of chemiluminescent labeling compounds include luminol, isoluminol, an aromatic acridinium ester, an imidazole, an acridinium salt and an oxalate ester. Similarly, a bio luminescent compound can be used to label the polypeptide of the present invention. Bioluminescence is a type of chemiluminescence found in biological systems in which a catalytic protein increases the efficiency of the chemiluminescent reaction. The presence of a bioluminescent protein is determined by detecting the presence of luminescence. Bioluminescent compounds that are useful for labeling include luciferin, luciferase and aequorin. Typically, when the polypeptide is fused to a fluorescent polypeptide as described above, the presence of the fusion protein can be detected with any means well known in the art such as a microscope or microscope or automated analysis system. Typically, when the polypeptide is fused to an enzyme then, the fusion protein is incubated in the presence of the appropriate substrate, the enzyme moiety reacts with the substrate to produce a chemical moiety which can be detected, for example, by spectrophotometric, fluorometric or visual means. Examples of enzymes that can be used to detectably label polyspecific immunoconjugates include β-galactosidase, glucose oxidase, peroxidase and alkaline phosphatase. Those of skill in the art will know of other suitable labels which can be employed in accordance with the present invention. The binding of marker moieties to anti-the polypeptide of the present invention is accomplished using standard techniques known to the art. Typical methodology in this regard is described by Kennedy et al., Clin. Chim. Acta 70: 1, 1976; Schurs et al., Clin. Chim. Acta 81 : 1, 1977; Shih et al., Int'U. Cancer 46: 1101, 1990; Stein et al, Cancer Res. 50: 1330, 1990; and Coligan, supra. Moreover, the convenience and versatility of immunochemical detection can be enhanced by using single domain antibodies of the present invention (fused or not to the heterologous polypeptide) that have been conjugated with avidin, streptavidin, and biotin. {See, e.g., Wilchek et al. (eds.), "Avidin-Biotin Technology," Methods In Enzymology (Vol. 184) (Academic Press 1990); Bayer et al., "Immunochemical Applications of Avidin-Biotin Technology," in Methods In Molecular Biology (Vol. 10) 149- 162 (Manson, ed., The Humana Press, Inc. 1992).) In some embodiments, the presence of the polypeptide (fused or not to the heterologous polypeptide) is detected with a secondary antibody that is specific for the single antibody of the present invention (fused or not to the heterologous polypeptide). Typically said secondary is labeled by same methods as described above. For instance when the polypeptide of the present invention is fused to a tag (e.g. histidine tag) the secondary antibody is specific for said tag. Methods for performing immunoassays are well-established. {See, e.g., Cook and Self, "Monoclonal Antibodies in Diagnostic Immunoassays," in Monoclonal Antibodies: Production, Engineering, and Clinical Application 180-208 (Ritter and Ladyman, eds., Cambridge University Press 1995); Perry, "The Role of Monoclonal Antibodies in the Advancement of Immunoassay Technology," in Monoclonal Antibodies: Principles and Applications 107-120 (Birch and Lennox, eds., Wiley-Liss, Inc. 1995); Diamandis, Immunoassay (Academic Press, Inc. 1996).).

In some embodiments, the polypeptide of the present invention is biotinylated. As used herein, the term "biotinylation" refers to the covalent binding of biotin to a polypeptide. The biotinylation of the polypeptide of the present invention is carried out using reagents capable of conjugating biotin to the side chain of said polypeptide, wherein said conjugation fundamentally takes place in the primary amino groups and in the thiol groups appearing in the side chains of the polypeptide. Suitable reagents for the biotinylation of amino groups include molecules containing biotin and a group capable of reacting with amino groups such as succinimide esters, pentafluorophenyl ester or alkyl halides, the biotin group and the reactive group being separated by a spacer of any length (for example, of 8-40 A in length). Some examples of these biotinylation agents include NHS-biotin agents (containing an ester bond of five carbon atoms between the biotin and the NHS group), sulfo-NHS-biotin, NHS-LC-biotin, sulfo-NHS-LC-Biotin, NHS-LC-LC-biotin, sulfo-NHS-LC-LC-biotin, sulfo-NHS-SS-biotin, NHS-PEO4-biotin, PFP-biotin, TFP-PEO-biotin and the like, wherein "NHS" indicates a N-hydroxysuccinimide group, "LC" refers to an amide type bond of 6 carbon atoms located between the NHS group and the biotin, "PEO" refers to a ethylene oxide group, wherein the subscript indicates the number of PEO units, "PFP" refers to a pentafluorophenyl group, "TFP" refers to a tetrafluorophenyl group, "sulfo" refers to a sulfonate group (SO3" Na+) and "SS" refers to a disulfide group. Examples of biotinylation reactive agents with thiol groups include molecules comprising biotin and a group of the maleimido or alkyl halide type, separated by a spacer of any length. Examples of biotinylation reagents include maleimide-PEG-biotin, biotin-BMCC (containing an N-terminal maleimido group and a cyclohexyl group, 2 amide bonds and 9 linking carbon atoms), PEO-iodoacetyl biotin, iodoacetyl-LC-biotin, biotin-HPDP (containing a pyridyl disulfide group) and the like.

In some embodiments, the polypeptide of the present invention is conjugated to a latex particle, a metal colloid particle, or a carbon nanotube.

One further object of the present invention relates to a method for detecting the presence of the *E coli* O25b-ST131 clone in a sample comprising i) contacting the sample with the polypeptide of the present invention which is capable of forming complexes with the lipopolysaccharide (LPS) molecules of the *E coli* O25b-ST131 clone and ii) detecting the presence of the one or more said complexes wherein the presence of at least one complex indicates the presence of the clone in the sample.

As used herein the term "sample" encompasses a variety of sample types obtained from a subject and can be used in an assay and susceptible to contain the *E coli* O25b-ST131 clone. Biological samples include but are not limited to blood and other liquid samples of biological origin, solid tissue samples such as a biopsy specimen or tissue cultures or cells derived therefrom, and the progeny thereof. In some embodiments, the sample is a liquid sample. In some embodiments, the liquid sample is urine, blood, serum, blood products, plasma, saliva, body fluid, water, culture medium, diluted culture medium, petroleum product, fuel, liquid undergoing fermentation, or a beverage. In some embodiments, the sample is a solid sample. In some embodiments, the solid sample is human or animal tissue, stool, sputum, expectorate, an agricultural product, food, solids collected by centrifugation or filtration, soil, or sediment. The sample may be diluted, purified, concentrated, filtered, dissolved, suspended or otherwise manipulated prior to assay.

The detection of the complexes may be performed by any well known method in the art and typically include immunoassays. Immunoassay formats that can be used typically include an enzyme-linked immunosorbent assay (ELISA), an immunofluorescence assay (IFA), a radioimmunoassay (RIA), a chemiluminescence immunoassay (CLIA), a lateral flow chromatographic test, a Western blot, an immunoprecipitation assay, flow cytometry, or fluorescence microscopy. Lateral flow immunochromatographic tests are particularly suitable for the rapid detection of the clone. Those skilled in the art also know that such assays may be useful for any of a large number of clinical microbiology problems, and other types of clinical samples. For performing said immunoassays the polypeptide is thus typically immobilised on a solid support.

Accordingly in some embodiments, the polypeptide of the present invention is immobilized on a solid support. In some embodiments, the solid support is a particle, a bead, a plastic or glass surface, a porous membrane, an array, or a chip. In some embodiments, the solid support forms part of an assay device. Solid phase assays, in general, are easier to perform than heterogeneous assay methods which require a separation step, such as precipitation, centrifugation, filtration, chromatography, or magnetism, because separation of reagents is faster and simpler.

In some embodiments, the present invention provides devices that are useful to detect and/or visualize the presence of the *E coli* O25b-ST131 clone in a sample. These devices may comprise a surface and the polypeptide of the present invention. Solid-phase assay devices include microtiter plates, flow-through assay devices (e.g., lateral flow immunoassay devices), dipsticks, and immunocapillary or immunochromatographic immunoassay devices. A particularly useful assay format is a lateral flow immunoassay format. In some embodiments, the device includes a solid support that contains a sample application zone and a capture zone. The lateral flow immunoassay (LFA) is a particular embodiment that allows the user to perform a complete immunoassay within 10 minutes or less. Those skilled in the art know many embodiments and variations of the lateral flow format, including: a variety of porous materials including nitrocellulose, polyvinylidene difluoride, paper, and fiber glass; a variety of test strip housings; colored and fluorescent particles for signal detection including colloidal metals, sols, and polymer latexes; a variety of labels, binding chemistries, and other variations. Various known formats exist for immunochromatographic test strips for detecting analytes in liquid samples. One format of LFA uses a direct binding "sandwich" assay, wherein the analyte is bound by two specific binding molecules which can thus include one polypeptide of the present invention. In some embodiments, the polypeptide of the present invention is fused to Fc domain (as described above) to form a antibody-like molecule than can be attached to a solid support by any method well known in the art. Examples of LFA format are described in U.S. Pat. No. 4,861,711; H. Friesen et al. (1989), which discloses a solid-phase diagnostic device for the determination of biological substances; U.S. Pat. No. 4,740,468; L. Weng et al. (1988) which discloses a solid phase specific binding method and device for detecting an analyte; U.S. Pat. No. 4,168,146; A. Grubb et al. (1979) which discloses a solid phase method and strip with bound antibodies and U.S. Pat. No. 4,435,504; R. Zuk (1984) which discloses a chromatographic immunoassay employing a ligand-binding molecule and a label conjugate. In one type of this format, described in U.S. Pat. No. 4,959,307; J. Olson (1990), the result is revealed as two lines (positive result) or one line (negative result). Another particularly useful assay format is a flow-through immunoassay format. Flow-through immunoassay devices involve a capture reagent (i.e. the polypeptide of the invention) bound to a porous membrane or filter to which a liquid sample is added. As the liquid flows through the membrane, target analyte (i.e. LPS molecules) binds to the capture reagent. The addition of sample is followed by (or made concurrent with) addition of detector reagent, such as labeled (e.g., gold-conjugated or colored latex particle-conjugated protein). Alternatively, the detector reagent may be placed on the membrane in a manner that permits the detector to mix with the sample and thereby label the analyte. The visual detection of detector reagent provides an indication of the presence of target analyte in the sample. Representative flow-through assay devices are described in U.S. Pat. Nos. 4,246,339; 4,277,560; 4,632,901; 4,812,293; 4,920,046; and 5,279,935; U.S. Patent Application Publication Nos. 20030049857 and 20040241876; and WO 08/030,546. Migration assay devices usually incorporate within them reagents that have been attached to colored labels, thereby permitting visible detection of the assay results without addition of further substances. See, for example, U.S. Pat. No. 4,770,853; PCT Publication No. WO 88/08534 and European Patent No. EP-A 0 299 428. There are a number of commercially available lateral flow type tests and patents disclosing methods for the detection of large analytes (MW greater than 1,000 Daltons). U.S. Pat. No. 5,229,073 describes a semiquantitative competitive immunoassay lateral flow method for measuring plasma lipoprotein levels. This method utilizes a plurality of capture zones or lines containing immobilized antibodies to bind both the labeled and free lipoprotein to give a semi-quantitative result. In addition, U.S. Pat. No. 5,591,645 provides a chromatographic test strip with at least two portions. The first portion includes a movable tracer and the second portion includes an immobilized binder capable of binding to the analyte. Additional examples of lateral flow tests for large analytes are disclosed in the following patent documents: U.S. Pat. Nos. 4,168,146; 4,366,241; 4,855,240; 4,861,711; and 5,120,643; European Patent No. 0296724; WO 97/06439; WO 98/36278; and WO 08/030,546. Devices described herein generally include a strip of absorbent material (such as a microporous membrane), which, in some instances, can be made of different substances each joined to the other in zones, which may be abutted and/or overlapped. In some examples, the absorbent strip can be fixed on a supporting non-interactive material (such as nonwoven polyester), for example, to provide increased rigidity to the strip. Zones within each strip may differentially contain the specific binding partner(s) and/or other reagents required for the detection and/or quantification of the particular analyte being tested for, for example, one or more molecules disclosed herein. Thus these zones can be viewed as functional sectors or functional regions within the test device. In general, a fluid sample is introduced to the strip at the proximal end of the strip, for instance by dipping or spotting. The fluid migrates distally through all the functional regions of the strip. The final distribution of the fluid in the individual functional regions depends on the adsorptive capacity and the dimensions of the materials used.

Lateral flow devices are commonly known in the art. Briefly, a lateral flow device is an analytical device having as its essence a test strip, through which flows a test sample fluid that is suspected of containing an analyte of interest. The test fluid and any suspended analyte can flow along the strip to a detection zone in which the analyte (if present) interacts with a capture agent (i.e. the polypeptide of the present invention) and a detection agent to indicate a presence, absence and/or quantity of the analyte. Numerous lateral flow analytical devices have been disclosed, and include those shown in U.S. Pat. Nos. 4,313,734; 4,435,504; 4,775,636; 4,703,017; 4,740,468; 4,806,311; 4,806,312; 4,861,711; 4,855,240; 4,857,453; 4,943,522; 4,945,042; 4,496,654; 5,001,049; 5,075,078; 5,126,241; 5,451,504; 5,424,193; 5,712,172; 6,555,390; 6,258,548; 6,699,722; 6,368,876 and 7,517,699; EP 0810436; and WO 92/12428; WO 94/01775; WO 95/16207; and WO 97/06439. Many lateral flow devices are one-step lateral flow assays in which a biological fluid is placed in a sample area on a bibulous strip (though non-bibulous materials can be used, and rendered bibulous, e.g., by applying a surfactant to the material), and allowed to migrate along the strip until the liquid comes into contact with a specific binding partner (such as an antibody) that interacts with an analyte (such as one or more molecules) in the liquid. Once the analyte interacts with the binding partner, a signal (such as a fluorescent or otherwise visible dye) indicates that the interaction has occurred. Multiple discrete binding partners (such as the polypeptide of the present invention) can be placed on the strip (for example in parallel lines) to detect multiple analytes (such as two or more molecules) in the liquid. The test strips can also incorporate control indicators, which provide a signal that the test has adequately been performed, even if a positive signal indicating the presence (or absence) of an analyte is not seen on the strip. The construction and design of lateral flow devices is very well known in the art, as described, for example, in Millipore Corporation, A Short Guide Developing Immunochromatographic Test Strips, 2nd Edition, pp. 1-40, 1999, available by request at (800) 645-5476; and Schleicher & Schuell, Easy to Work with BioScience, Products and Protocols 2003, pp. 73-98, 2003, 2003, available by request at Schleicher & Schuell BioScience, Inc., 10 Optical Avenue, Keene, N.H. 03431, (603) 352-3810.

In some embodiments, the method of the present invention is particularly suitable in diagnostic assays, in particular for the diagnosis of bacterial infections caused by the *E coli* O25b-ST131 clone. In some embodiments, the method of the present invention is particularly suitable for the diagnosis nosocomial infections and in particular, Hospital-acquired nosocomial infections. In some embodiments, the method of the present invention is particularly suitable for the diagnosis of an infectious disease selected from the group consisting of cystic fibrosis, otitis media, keratitis, endophthalmitis, bacteremia, burn wound infection, pneumonia, meningitis, peritonitis or sepsis, more preferably pneumonia, meningitis, peritonitis or sepsis, and most preferably peritonitis or sepsis. Accordingly, in some embodiments, the sample is a sample obtained from a patient who suffers from a bacterial infection. In some embodiments, the patient is selected among immunocompromised and/or seriously ill patients in cancer centers, intensive care units, and organ transplant centres. In some embodiments, the diagnostic method of the present invention is a valuable tool for practicing physicians to make quick treatment decisions. These treatment decisions can include the administration of an antibacterial agent (e.g. antibiotic) and decisions to monitor a subject for onset and/or advancement of the infection. The method disclosed herein can also be used to monitor the effectiveness of a therapy. The patient can be monitored while undergoing treatment using the methods described herein in order to assess the efficacy of the treatment protocol. In this manner, the length of time or the amount give to the patient can be modified based on the results obtained using the methods disclosed herein.

The invention will be further illustrated by the following figures and examples. However, these examples and figures should not be interpreted in any way as limiting the scope of the present invention.

### FIGURES:

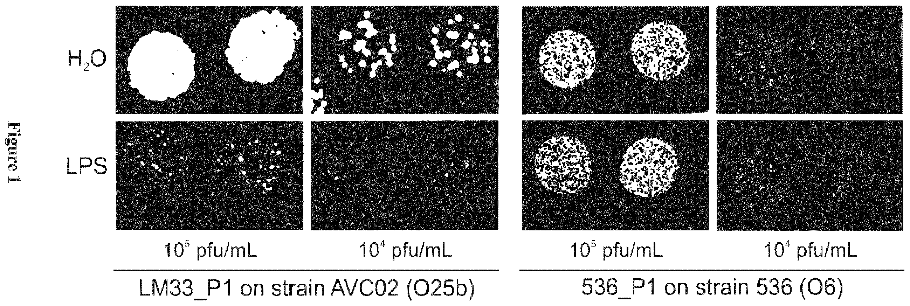

**Figure 1****. O25b LPS extract inhibits bacteriophage LM33_P1 infection: appearance on agar plates.** LPS extract from strain LM33 was mixed with bacteriophage LM33_P1 (left) or 536_P1 (right) at two different concentrations (10⁵ and 10⁴ pfu/mL) and assayed on two agar plates overlaid with an O25b strain (AVC02) or an O6 strain (536) as control. Enlargements of these two plates are shown to facilitate the observation.

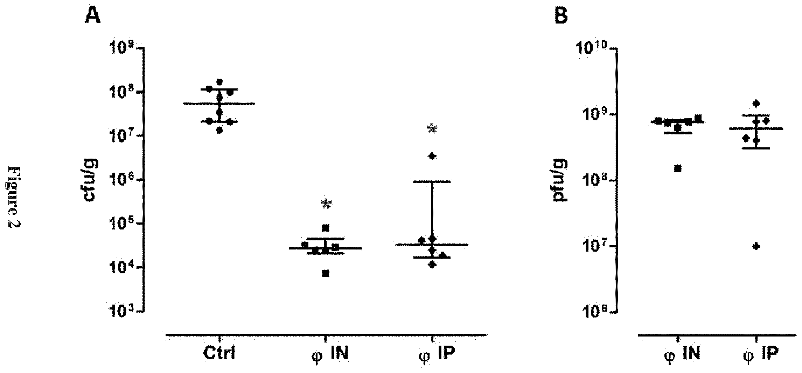

**Figure 2****. Bacteriophage LM33_P1 *in vivo* activity in a lung infection model.** Bacterial (A) and viral (B) counts 17 hours post-infection in lungs homogenate of mice infected with 1x10⁸ cfu of strain LM33. Four hours post-infection, mice received either PBS (Ctrl, n=8, intranasally and intraperitoneally) or bacteriophage LM33_P1 by intranasal route (φ IN, MOI 50, n=6) or by intraperitoneal route (φ IP, MOI 500, n=6). Results are expressed as individual values with median and interquartile ranges (25^{th} and 75^{th} percentiles). *: p <0.001 compared to control group.

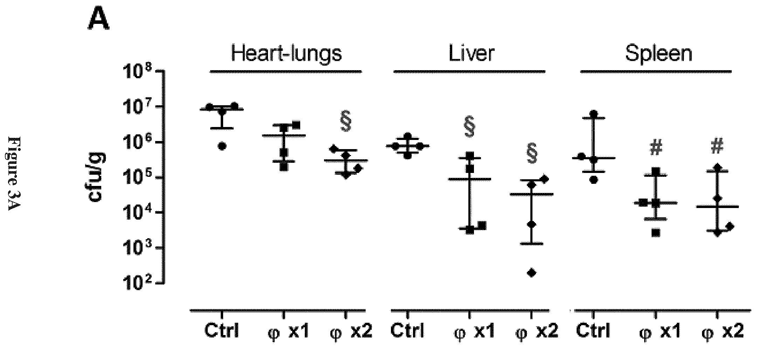

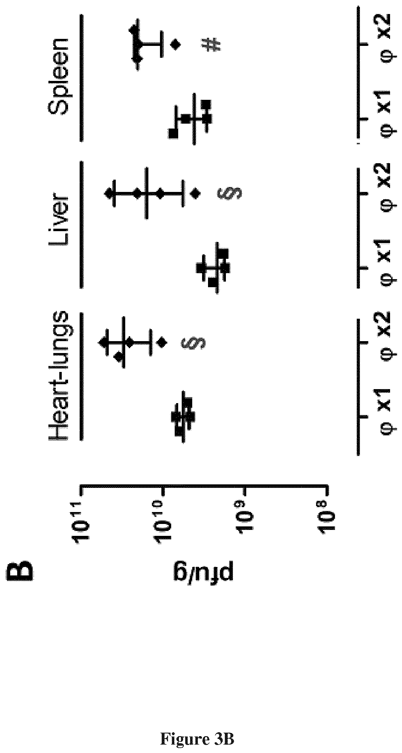

**Figure 3****. Bacteriophage LM33_P1 *in vivo* activity in a septicemia model.** Bacterial (A) and viral (B) counts 20 hours post-infection in indicated organs of mice infected with 1x10⁹ cfu of strain H1659 (ST131-O25b:H4). Two hours post-infection, mice received intraperitoneally either PBS (Ctrl) or bacteriophage LM33_P1 at a MOI of 60 (φ X1: one dose 2 hours post-infection, φ X2: two doses 2 and 12 hours post-infection). Results are expressed as individual values (4 animals per condition) with median and interquartile ranges (25^{th} and 75^{th} percentiles). §: p <0.05 compared to control group, #: p =0.057 compared to control group.

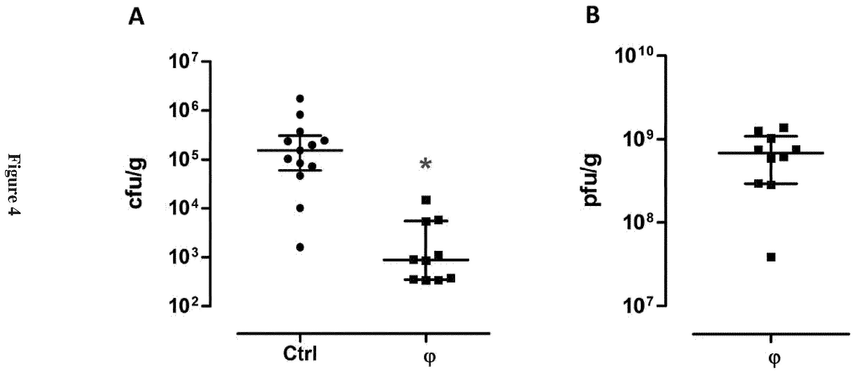

**Figure 4****. Bacteriophage LM33_P1 *in vivo* activity in a urinary tract infection model**. Bacterial (A) and viral (B) counts 48 hours post-infection in kidneys homogenates of mice infected with 5x10⁷ cfu of strain LM33. Twenty four hours post-infection, mice received intraperitoneally either PBS (Ctrl, n=13) or bacteriophage LM33_P1 (φ, MOI 200, n=10). Results are expressed as individual values with median and interquartile ranges (25^{th} and 75^{th} percentiles). *: p <0.001 compared to control group.

### EXAMPLE:

### Material & Methods

### Bacterial strains and bacteriophages, susceptibility testing

Bacterial strains used in this work belong to previously published collections of human commensal and extraintestinal *E. coli* gathered in France during the 2010s (13-15), from the ECOR collection (16) and the ColoColi collection (an ongoing French multicenter study collecting *E. coli* strains in the lower respiratory tract of mechanically ventilated patients). Phylogroup and ST belonging was determined as described in (17, 18). O-type and *fimH* allele were determined by PCR-based assays as previously described (19, 20), respectively. All strains were grown in lysogeny broth (LB) (Difco™ Bacto-Tryptone 10 g/L, Difco™ Yeast extract Difco 5 g/L, NaCl 5 g/L). Antibiotic susceptibility using the disk diffusion method was performed following the guidelines of the European Committee for Antimicrobial Susceptibility Testing guidelines.

Some *E. coli* strains, used for lipopolysaccharide (LPS) assays or bacteriophage susceptibility testing, are detailed below:
LM33, LM36, AVC02 (ST131-O25b:H4) and AVC03 (O25b, non-ST131) are clinical strains responsible for ventilator-associated pneumonia,
536 (ST127-O6), LM02 (ST69-O17) and ECOR51 (ST73-O25a) have been used as source of their corresponding LPS,
81009 WT (ST131-O25b:H4) and its isogenic rough derivative (mutant strain obtained by deleting the gene encoding for the O-antigen ligase) (21) were used to prove the LPS-dependent interaction of LM33_P1.

Bacteriophages were isolated from sewage, using specific host. By convention, bacteriophages are named as follows: "host bacteria_Px" (for example LM33_P1 represents the first bacteriophage isolated using strain LM33). In all competition experiments, bacteriophage solutions were purified using ultracentrifugation on cesium chloride gradient as previously described (22).

For bacteriophage susceptibility testing, we used double spot test (23) as screening method to identify resistant strains. Briefly, spot test consisted in dropping off 10 µL of a growing liquid culture of the bacterial strain (OD₆₀₀ₙₘ 0.5) on an agar plate. After drying, 1 µL of the bacteriophage solution (LM33_P1, 10⁷ pfu/mL) was added on one half of the bacterial drop. Plate was then incubated at 37°C during 4 hours before reading. A susceptible strain was identified by the presence of a crescent-shaped lysis area on the bacterial drop or the visualization of individualized plaques. Efficiency of plaquing (EOP) was determined for all susceptible strains by titrating the solution of LM33_P1 on both its host (LM33) and the evaluated strain. EOP was calculated as the ratio of number of plaques formed by the bacteriophage on the non-host strain to the number of plaques formed on its host, using the same bacteriophage solution. Only strains for which individualized plaques were observed were considered as susceptible strains. For strain 81009 WT and its rough derivative mutant, tests were performed at 20 °C to turn-off type II capsule expression (24).

### LPS extraction

LPS extracts were purified from the same amount of bacteria (10¹⁰ cfu) using a hot phenol-water-diethyl ether extraction (25) followed by extensive dialysis against sterile pyrolyzed water. High purity LPS was confirmed by performing agarose gel electrophoresis with ethidium bromide staining (nucleic acids detection) and SDS-PAGE 12% followed by Coomassie blue staining (proteins detection). Ten µL of each LPS extract were migrated on a SDS-PAGE 10% followed by silver staining to visualize the LPS O-antigen pattern (SilverSNAP Stain Kit II, Pierce).

### Plaques inhibition assays with LPS extracts

From purified stock solution of bacteriophages in TN buffer (Tris-HCl 10 mM, NaCl 150 mM, pH 7.5), 3 solutions of 10⁶, 10⁵ and 10⁴ pfu/mL in TN buffer were prepared. Each of these working solutions was used to prepare final tubes with bacteriophages alone (100 µL of working solution + 100 µL of pyrolyzed water) and tubes with bacteriophages + LPS (100 µL + 100 µL of undiluted LPS extract). Additional tubes containing bacteriophages and decreasing amounts of LPS were also prepared (pyrolyzed water was used to reach an identical final volume). Then, 10 µL of each final bacteriophage tubes, with and without LPS, were spotted in triplicate on an agar plate, previously overlaid by the bacteria to test. Plates were incubated during 4 hours at 37°C before plaques-forming units were numerated in each condition.

### Characterization of bacteriophage LM33_P1

Adsorption assay and one-step growth were performed using LB (Difco™ Bacto-Tryptone 10 g/L, Difco™ Yeast extract Difco 5 g/L, NaCl 5 g/L), under constant shaking (100 rpm) at 37°C, as described by Hyman and Abedon (26), in triplicate. A correlation curve was extrapolated from raw data using nonlinear regressions (GraphPad Prism 5.0, GraphPad software, California): a dose-response model was used for one step growth experiment (Y=Bottom + (Top-Bottom)/(1+10^{∧}((LogEC50-X)^{∗}HillSlope)) with Y=log(pfu/infected cell) and X=time) and an exponential model with one phase decay for adsorption experiment (Y=(Y0 - Plateau)^{∗}exp(-K^{∗}X) + Plateau with Y=free phages(%), X=time). Growth parameters (eclipse and latent period, burst size) were then derived from these regressions. Adsorption constant was calculated as -p/N where p is the slope of the straight line obtained after a natural logarithm transform and N the concentration of bacteria when starting the adsorption assay.

### Lysis kinetic (with and without LPS extracts) and aggregation assays with O25 antibody

Lysis kinetics were performed as detailed in the SI. Briefly, the growth of LM33 with and without LM33_P1 was followed overtime by recording optical density at 600 nm every 15 minutes.

Aggregation assays were performed using O25 *E. coli* anti-serum (Statens Serum Institut, Copenhagen, Denmark) and observed under light microscope as detailed in the SI.

### Sequencing of the strain LM33 and bacteriophage LM33_P1

Sequencing of bacteriophage LM33_P1 and strain LM33 was performed using Illumina sequencing technology (Illumina Inc., San Diego, CA). LM33_P1 DNA was extracted from a purified bacteriophage solution, using DNase and RNase pretreatments followed by a phenolchloroform extraction, modified from Pickard (27). LM33 genomic DNA was extracted using a MaxWell Tissue DNA Purification kit (Promega, Madison, WI). Genomes annotation was performed by MicroScope plateform for strain LM33 and with RAST server for bacteriophage LM33_P1 (28, 29) followed by manual curation.

### Murine experimental infections models

Animal were housed in animal facilities in accordance with French and European regulations on the care and protection of laboratory animals. Protocols were approved by the veterinary staff of the Institut Pasteur and INSERM animal facilities as well as the National Ethics Committee regulating animal experimentation. Food and drink were provided *ad libitum.*

Pneumonia was initiated by intranasal administration of 1x10⁸ cfu of strain LM33 on anesthetized eight-week-old 25 g BALB/cJRj male mice (Janvier, Le Genest Saint Isle, France) as previously described (30). Mice were treated using bacteriophage LM33_P1 four hours post-infection, either by using the intranasal route (multiplicity of infection of 50, *i.e.* a ratio of viruses to bacteria equal to 50) or the intraperitoneal route (MOI of 500). Control mice received accordingly an intranasal or intraperitoneal identical volume of PBS (phosphate-buffered saline). Lungs were collected 17 hours post-infection on euthanized animals.

The septicemia model, as previously described, is essentially used to study intrinsic extraintestinal virulence of *E. coli* isolates (7). Four-week-old 17 g OF1 female mice (Janvier, Le Genest Saint Isle, France) were injected subcutaneously into the nape of the neck with 1x10⁹ cfu of strain H1659 (ST131-O25b:H4) (6). Because of the high inoculum used, we tested both a single and a double dose of bacteriophages: the single dose (MOI 60) was administered by intraperitoneal injection 2 hours post-infection while the double dose consisted in an injection (MOI 60) administered 2 and 12 hours post-infection. Control mice received an identical volume of PBS. Organs targeted by septic metastasis (heart-lung, spleen and liver) were collected on animals that died between 24 to 30 hours post-infection.

The urinary tract infection model consists in a retrograde kidneys infection occurring after an intra-urethral injection of 5x10⁷ cfu of strain LM33 in the bladder, as previously described (31). Twenty-four hours after infection, 8-week-old 17 g CBA/j female mice (Charles River, Chatillon-sur-Chalaronne, France) were treated intraperitoneally with LM33_P1 (MOI of 200) while control mice received an identical volume of PBS. Kidneys were collected 48 hours post-infection.

In all cases, organs were mechanically homogenized in cold PBS using a gentleMACS Octo Dissociator (Milteny Biotec, Bergisch Gladbach, Germany) before being serially diluted and spread on Drigalski agar plates containing appropriate antibiotic to numerate colony. Bacteriophages count was performed on supernatant after centrifugation of homogenates according to routine methods.

### Statistical analysis

All statistical analyses were performed by using GraphPad Prism version 5.00 (GraphPad Software, La Jolla, CA). The normal distribution of all variables was checked using the Kolmogorov-Smirnov test, and results are then expressed as mean ± SD. In case of non-Gaussian distribution, results are expressed as median [25th, 75th percentile]. Statistical tests (Student t test or Mann-Whitney test) were chosen accordingly.

### Results:

**Bacteriophage LM33_P1 targets antibiotic resistant O25b *E. coli* strains.** The *E*. *coli* strain LM33 (isolated from an intensive care unit patient who developed a ventilator-associated pneumonia) was used to isolate bacteriophage LM33_P1. Strain LM33 displays an O25b:H4 serotype, a B2 phylogroup (subgroup I) and a ST131 sequence-type as well as a multi-drug resistance phenotype with an extended spectrum beta-lactamase, a resistance to nalidixic acid, aminoglycosides (kanamycin, tobramycin, gentamicin, netilmicin excepted for amikacin where an intermediate phenotype is found), sulphonamides and chloramphenicol. The beta-lactam resistance is supported by a plasmid (pLM33) bearing the *blaTEM-1c* (penicillinase) and *blaSHV-12* (extended spectrum beta-lactamase) genes, as well as by the bacterial chromosome containing the *blaDHA-7* gene encoding a cephalosporinase and also a copy of the *blaSHV-12* and *blaTEM-1c* gene (**Table 1**).

We determined the host range of bacteriophage LM33_P1 on a panel of 283 *E. coli* strains belonging to various O-types (**data not shown**). One hundred and eighty-three (64%) of these strains were not O25b and none of them was infected by LM33_P1, including twelve O25a strains and six ST131-O16 strains. Among the remaining one hundred O25b strains (encompassing 83 ST131, 4 ST69, 10 ST95 and 3 others STs), 64 (64%) were infected by LM33_P1 with a median efficiency of plaquing of 0.46 [0.09-1.27]. Interestingly, LM33_P1 was found to be more efficient on STs associated with high antibiotic resistance (ST131 and ST69) where 70% of these strains were lysed while it was weakly efficient on ST associated with low antibiotic resistance (ST95 and others) where only 23% of these strains were susceptible (**data not shown**). Finally, we did not find a correlation between susceptibility to bacteriophage LM33_P1 and the *fimH* allele H30, which is strongly associated with fluoroquinolone resistance among ST131 strains (32).

**Bacteriophage LM33_P1 is a *Podoviridae* distantly related to bacteriophage T7.** Genome of bacteriophage LM33_P1 (38 979 bp; GC content of 50.8%; 49 ORFs predicted) lacks putative ORFs with homologies to integrase or recombinase.

A BLAST analysis of the genomic sequence revealed that the four closest related bacteriophages were enterobacteria bacteriophages: three coliphages called PE3-1, K1F (33), EcoDS1 (with 94% identity on ≥ 88% of its length for all of them) and bacteriophage Dev2 infecting *Cronobacter turicensis* (with 83% identity on 85% of its length) (34). Alignment of these related bacteriophages with LM33_P1 revealed a similar spatial genome organization and confirmed the high homology between them (**data not shown**). Strikingly, the 5' extremity (the first 650 nucleotides) of the tail fiber gene is highly conserved in each bacteriophage genome, while the remaining part is highly divergent. The corresponding N-terminal region (IPR005604 / PF03906, InterPro / Pfam database) of this tail fiber protein is involved in its connection to the tail-tube (35) while the C-terminal part, involved in host recognition, often carries hydrolase activities as the endosialidase of bacteriophage K1F used for exopolysaccharide degradation (33, 36). BLAST searches on the C-terminal part of the tail fiber of bacteriophage LM33_P1 revealed homology to a domain belonging to the pectin lyase superfamily (IPR011050). Tridimensional structure prediction using Phyre² database (37) confirmed its close proximity to the endopolygalacturonase of *Erwinia carotovora* that belongs to the pectin lyase superfamily (100% amino-acid predicted with a confidence >90% for the tertiary structure, index of confidence for homologous protein 94.1%, Protein Data Bank entry: 1BHE).

**Bacteriophage LM33_P1 is highly efficient and rapid** ***in vitro.*** Adsorption of LM33_P1 bacteriophage on its host is fast with ≥ 90% of the viral population attached to cells after 3.5 minutes with an adsorption constant of 1.2x10⁻⁸ mL/min. Newly produced virions are detected within the bacteria as soon as 7 minutes post-infection (eclipse period) while host lysis occurs in 9 minutes (latent period) with a burst size of 317 (95% confidence interval: 289-345) (**data not shown**).

In liquid medium, when LM33_P1 was mixed with its host, the absorbance value of LM33 cells started to decline (sign of lysis) within 15 minutes (MOI of 1). With much fewer bacteriophages (MOI of 10⁻⁶) lysis still occurred within 60 minutes. On solid medium, LM33_P1 forms clear and large plaques, whose diameter increases rapidly overtime with a visible halo around clear areas. This halo suggests the presence of a diffusible enzyme that most likely carries a depolymerase activity (38).

**Bacteriophage LM33_P1 specifically binds to O25b LPS O-antigen.** Host range of bacteriophage LM33_P1 strongly suggested that O-chain of LPS could be involved in its specificity. Using LPS competition assays we observed that purified LPS from strain LM33 was able to partially inhibit interaction between bacteriophage LM33_P1 and strain LM33 as well as other O25b strains (**Table 2**).

First, we demonstrated that purified LPS reduced the number of plaque-forming units when mixed with bacteriophages before application on a bacterial layer (mean reduction of 1.0 ±0.23 Log₁₀ from 15 assays with five different O25b strains). Together with the reduction of the number of plaques, we observed a reduction of plaque diameters suggesting that LPS molecules prevented newly released bacteriophages to infect surrounding hosts (**Figure 1**). These observations are specific of bacteriophage LM33_P1 interaction with O25b strains since: i) O25b LPS extract from strain LM33 was not able to affect interaction of other bacteriophages targeting non O25b strains and ii) LPS extract from non O25b strains (O25a, O6 and 017) was unable to alter interaction between bacteriophage LM33_P1 and strain LM33 (**Table 2**).

Second, LPS extract from O25b strain (LM33) was also reducing infectivity of bacteriophage LM33_P1 on liquid medium in a dose dependent manner (**data not shown**), while LPS extracts from O6 and O25a strains had no effect.

Third, using an O-type specific antibody to aggregate O25 strains for serotyping, we found that bacteriophage LM33_P1 prevented aggregation of strain LM33 (**data not shown**).

Fourth, using the *E. coli* O25b 81009 and its isogenic rough derivative (LPS deficient strain obtained by deleting the gene encoding for the O-antigen ligase) (21) we observed that bacteriophage LM33_P1 infects the wild type strain 81009 while the LPS deficient strain is resistant. Conversely, we confirmed that bacteriophage LM33_P1 could not adsorb on the LPS defective strain.

**Adsorption of bacteriophage LM33_P1 is hindered by capsule production.** Production of exopolysaccharides is a well-known bacteriophage resistance mechanism and might be involved in the non-adsorption of bacteriophage LM33_P1 observed in five randomly chosen LM33_P1 resistant strains (81009 WT, JJ1886, S242, B-1, C-1). Since, in some cases (type II capsule), the synthesis of exopolysaccharides is temperature dependent, we investigated LM33_P1 susceptibility on all O25b resistant strains (n=36) at 20°C. We observed that nine of them (25%) became susceptible at this temperature (**data not shown**).

**Bacteriophage LM33_P1 efficiently infects its host** ***in vivo.*** As bacteriophage LM33_P1 exhibited impressive *in vitro* characteristics, we investigated its *in vivo* activity in three different animal infection models relevant to ST131 clinical epidemiology: pneumonia, septicemia and urinary tract infection (**Figures 2-4**). Since strain LM33 was isolated from a patient with pneumonia, we first attempted to establish pneumonia in mice. Using an inoculum 50 times higher than previously reported in such model (30) and despite clear macroscopic lung lesions, strain LM33 was not lethal preventing us to use survival as an indicator of bacteriophage efficacy. We therefore evaluated LM33_P1 efficacy by counting bacteria from lung homogenates collected 17 hours following infection. Three groups of mice were treated 4 hours post-infection either by control solution (PBS), intranasal (MOI 50) or intraperitoneal (MOI 500) bacteriophages. Independently of the administration route, we observed a 3 Log₁₀ reduction in bacterial load when mice received bacteriophage treatments compared to control group (PBS-treated animal: 5.4x10⁷ cfu/g, intranasally LM33_P1-treated: 2.7x10⁴ cfu/g, intraperitoneally LM33_P1-treated: 3.3x10⁴ cfu/g, p <0.01). Interestingly, the number of bacteriophages in the lung tissue was similar between intranasally and intraperitoneally-treated mice despite the latter had received 10 times higher dose.

Then, we challenged the fast *in vitro* kinetics parameters of bacteriophage LM33_P1 in a murine model of septicemia previously reported (6, 7) using the H1659 ST131-O25b:H4 strain (6) (strain LM33 was not lethal in this model), on which LM33_P1 is as efficient as on strain LM33 (EOP = 1). Following a subcutaneous inoculation of 1x10⁹ cfu, septic metastasis in several organs were rapidly observed (first deaths occurred in less than 24 hours). Intraperitoneal administrations of bacteriophage LM33_P1 (MOI 60, single dose at H2 post-infection or two doses at H2 and H12 post-infection) were not sufficient to prevent animals death. However, in a subset of animals that died within the same time interval (between 24 and 30 hours), bacteria and bacteriophages content was analyzed: i) in liver, spleen and lung-heart homogenates of bacteriophage-treated groups the number of bacteria was reduced compared to control group; ii) two doses appeared to be more efficient than a single one, reaching a significant reduction of approximately 1.4 Log₁₀ (median bacterial count decrease from 8.5x10⁶ to 2.9x10⁵ in heart-lungs, 7.7x10⁵ to 3.2x10⁴ in the liver and 3.5x10⁵ to 1.4x10⁴ cfu/g in the spleen); iii) bacteriophage counts were in the same order of magnitude in all organs, but were significantly higher when two doses were administered (2.0x10¹⁰ vs 4.0x10⁹ pfu/g, p <0.01); iv) the amount of bacteriophages was 3 to 6 Log₁₀ higher than the amount of the bacteria in each mouse for all organs. All of these observations revealed that bacteriophage LM33_P1 was able to infect strain H1659 in each organ considered.

Finally, as *E. coli* is a major pathogen in UTIs, we assessed bacteriophage LM33_P1 efficacy in a murine UTI model. Twenty-four hours following intra-urethral injection of 5.10⁷ cfu of strain LM33, mice received a single bacteriophage treatment intraperitoneally (MOI of 200). Fourty-height hours post-infection, a 2 Log₁₀ reduction of the bacterial load was observed in the kidneys in the treated group compared to control (1.5x10⁵ vs 8.8x10² cfu/g, p <0.001).

Altogether these data firmly demonstrate the ability of bacteriophage LM33_P1 in infecting O25b strains *in vivo.*

### Discussion:

Antibiotic resistance is a public health problem worldwide. In less than 10 years, multi-drug resistant ST131-O25b:H4 *E. coli* clonal complex have spread over the planet, now being present in both animals and humans (2). Unfortunately, the discovery of new antibiotics did not turn out to be as successful as initially expected, leading to the reappraisal of phage therapy. One of the main advantages of bacteriophages often reported is their specificity to infect few strains within a species, having then a limited impact on patient's microbiota. Along with monoclonal antibodies (anti-O25b antibodies have been proven to exert a protective effect in mouse septicemia model) (39), bacteriophages are the only anti-infectious tools that could reach such specificity.

Using an ST131-O25b:H4 clinical isolate of *E. coli* (strain LM33), we isolated a bacteriophage, LM33_P1, which was found to be extremely specific. Extensive tests on almost 300 strains belonging to various serotypes revealed that this bacteriophage infects exclusively O25b strains. Interestingly, O25b O-antigen is present in the archetypal ST131 clonal complex but also in ST69, another antibiotic resistant spreading clone of *E. coli,* the "clonal group A" (11, 40). In a therapeutic projection and based on the pandemic lineages of extraintestinal pathogenic *E. coli* (41), we observed a greater susceptibility among both of these STs (70%) compared to less antibiotic resistant O25b STs like ST95 and minor ones (23%).

Additionally, the majority of strains belonging to the ST131 clonal complex displays an O25b O-antigen while a minor part, less resistant to antibiotics, displays an 016 serogroup (42). Bacteriophage LM33_P1 specificity was linked to the O25b O-antigen and not to the sequence type (*i.e.* none of the non-O25b ST131 strains were susceptible to bacteriophage LM33_P1 while all O25b-ST69 strains tested were susceptible). Furthermore, susceptibility of ST131-O25b:H4 strains to bacteriophage LM33_P1 was independent of the *fimH* allele, a marker of the epidemiologic evolution of this clone (32). Besides, bacteriophage LM33_P1 was unable to infect O25a strains, despite a highly similar O-antigen structure where polysaccharides repeated units only differ by one monosaccharide (fucose *versus* rhamnose), a fine discrimination that is not possible with classical antibodies used for serotyping until the recent description of O25b monoclonal antibodies (21).

Our investigations led to estimate that global host coverage of bacteriophage LM33_P1 on O25b strains is 64%. We consider that this coverage is reliable as we first avoided sampling bias by screening a large collection (maybe one of the largest ever tested for such study) obtained from different sources with many serotypes. Second, we assessed strain susceptibility in a rigorous way using EOP determination that excludes atypical results and false positive like lysis from without (43, 44). Finally, 90% of EOP values were within -1.5 and 1.5 Log₁₀ units, which indicate that strains infected with a very low efficiency are infrequent. In addition to this specialized host range, we found that bacteriophage LM33_P1 possesses optimized properties to infect its host. Compared to data available in the literature, we found that it is the quickest T7-like bacteriophage ever reported, lysing its host within 10 minutes while T7 takes 13 to 16 minutes (45, 46). Part of this success relies on its absorption constant (1.2x10⁻⁸ mL/min) which was found 10 times higher that most of bacteriophages (47-50) and its burst size that is also on the top half of values usually observed (51).

To prevent phage adsorption bacteria can mask phage receptors by the production of extracellular exopolysaccharides (capsules), which can also help bacteria escaping immune cells recognition (52, 53). We found that 25% of strains reversed their phenotype towards bacteriophage LM33_P1 from resistant to susceptible, when tested at 20°C, a temperature known to turn off type II capsule production (24). Therefore, bacteriophage LM33_P1 coverage increased to 80% among all ST131-O25b:H4 strains and to 73% among all O25b strains tested. It was also previously shown that bacteriophages can defeat this defense mechanism using tail fibers that possess depolymerase activities (54-57) and we can reasonably assume that isolation of LM33_P1 variants or different bacteriophages could provide such solution to improve (by synergy) the coverage rate of O25b strains (56, 58, 59).

With the goal of using bacteriophages to treat human bacterial infections, the translation from *in vitro* activity (forming plaques) to *in vivo* efficacy (curing a disease) is not guaranteed, despite high success rate (60). Our investigation of the *in vivo* curative potential of bacteriophage LM33_P1 revealed indeed that, in the three models tested, this bacteriophage was able to infect targeted bacteria in several body compartments and via different administration routes. These treatments were not optimized to reach maximum efficacy as many parameters would need to be evaluated, which require dedicated studies out of the focus of this work. Indeed, bacteriophages pharmacokinetic is highly complex, due to their intrinsic properties (bacteria-driven self-expansion, diffusion, adsorption, threshold to prime a viral expansion, *etc*.) (61-63) and cannot be compared to traditional pharmacokinetic of antibiotics. In addition, in such experimental models, the curative dose applied is always related to the initial known dose of pathogenic bacteria, which is therefore a gross estimation of what is needed (amount of bacteria could be highly different between time of inoculation and treatment due to bacterial growth). Consequently, our data should not be over-translated to the clinical setting. Nevertheless, it remains indisputable that bacteriophages, including LM33_P1 as shown in this study, can quickly reduce the load of their host within a complex environment including the gut of mammals (64). Our data also support higher efficacy when bacteriophages are applied locally (intranasal instillation to treat pneumonia) than when used via a systemic administration. In a therapeutic approach, such bacteriophages could be used as a selective antimicrobial agent for controlling passive carriage of ST131-O25b:H4 strains in human gut in order to reduce its dissemination, particularly in healthcare-associated environments. Indeed, *E. coli* strains residing in the digestive tract constitute a well-known reservoir for urinary tract infections but probably also for ventilator-associated pneumonia (14). Finally, as no positive correlation between antibiotic and bacteriophage resistance has ever been shown, phage therapy remains a valuable resource to control such multi-drug resistant pathogens. Clinical trials are now required and are indeed encouraged by the recent position taken by the European Medicine Agency (65), in order to better define to which extent promises of bacteriophages, such as the one reported here, can be translated into efficient treatment.

Beside the classical phage therapy approach, bacteriophage LM33_P1 or proteins from it offer opportunities to develop several tools. The tail fiber could be used to kill specifically O25b *E. coli* strains using bacteriocins, as previously shown for 0104 *E. coli* strains involved in enterohemorragic colitis (66). Other approaches could be foreseen where bacteriophages are reprogrammed and could suppress antibiotic resistance genes using CRISPR-Cas system (67) or express well-chosen beneficial enzymes to fight biofilm (68). Deeper investigations on the infectious cycle of this bacteriophage are now required to determine which molecular mechanisms are responsible for its fast-killing component. Bacteriophage LM33_P1 could also be used from now as a starting platform to develop highly virulent synthetic bacteriophages with various host specificity (69).

**Table 1. Main genotypic characteristics of strain LM33 and plasmid pLM33.**

| **Strain LM33 chromosome (accession number: PRJEB9970)** | | |
|---|---|---|
| **General informations** | | |
| Genome size: 5 450 287 bp | GC content: 51.5 % | Number of genes: 5276 |
| Sequence type: ST131 | Serotype: O25b:H4 | Phylogroup: B2 |
| (according to the Achtman scheme) | | *fimH* allele: 22 |

| **Genes coding for virulence factors*** | | |
|---|---|---|
| *iss* (increased serum survival) | | *aer* (aerotaxis sensor receptor) |
| *iroN* (Enterobactin siderophore receptor protein) | | *fyuA* (siderophore) |
| *prfB* (P-related fimbriae regulatory gene) | | *papC* (P fimbriae) |
| *traT* (serum resistance-associated outer membrane protein) | | *papGIII* (P fimbriae) |
| *gad* (glutamate decarboxylase) | | *mchF* (ABC transporter protein) |

| **Genes coding for antibiotic resistance*** | | |
|---|---|---|
| Aminoglycoside resistance: *strB*, *aacA4*, *strA*, *aac(6')-IIc* | | |
| Beta-lactam resistance: *blaDHA-7*, *blaSHV-12*, *blaTEM-1C* | | |
| Quinolone resistance: *aac(6')Ib-cr, qnrB4* | | |
| MLS resistance: *ere(A)* | | |
| Sulphonamide: *sull* ; thrimethoprim: *dfrA18* | | |
| | | |
| | | |

| **Plasmid pLM33 (accession number: PRJEB9970)** | | |
|---|---|---|
| **General informations** | | |
| Plasmid size: 296 909 bp | GC content: 47.2% | Number of genes: 382 |
| Incompatibility group: H | | |

| **Genes coding for virulence factors*** | | |
|---|---|---|
| none | | |

| **Genes coding for antibiotic resistance*** | | |
|---|---|---|
| Aminoglycoside resistance: *strA*, *strB*, *aacA4*, *aac(6')-IIc* | | |
| Beta-lactam resistance: *blaSHV-12, blaTEM-1C* | | |
| Quinolone resistance: *aac(6')Ib-cr* | | |
| MLS resistance: *ere(A)* | | |
| *data obtained using the center for genetic epidemiology server (70, 71) | | |
| | | |
| | | |

**Table 2: Data obtained during plaque test inhibition assays with different LPS extracts and randomly chosen couples of viruses-bacteria. (+)/(-): presence/absence of an inhibitory effect of LPS extract, - : not tested.**

| **Interaction tested** | | **Inhibitory effect of various LPS extracts** | | | |
|---|---|---|---|---|---|
| Bacteriophage | Bacteria (serotype) | O25b (LM33) | O6 (536) | 017 (LM02) | O25a (ECOR51) |
| LM33_P1 | LM33 (O25b) | (+) | (-) | (-) | (-) |
| " " | LM34 (O25b) | (+) | (-) | (-) | (-) |
| " " | LM36 (O25b) | (+) | (-) | (-) | (-) |
| " " | AVC02 (O25b) | (+) | (-) | (-) | (-) |
| " " | AVC03(O25b) | (+) | (-) | (-) | (-) |
| 536_P1^{a} | 536 (O6) | (-) | (-) | - | - |
| 423_P1^{b} | H17 (016) | (-) | - | - | - |
| 416_P1^{b} | LM49 (O2b) | (-) | - | - | - |
| LF82_P2^{c} | LF82 (O83) | (-) | - | - | - |
| LF82_P2^{c} | RY09 (O4) | (-) | - | - | - |

| | | | | | |
|---|---|---|---|---|---|
| ^{a} described in (30), ^{b} bacteriophages isolated using ventilator-associated pneumonia (VAP) strains (423, 416) and active on others VAP strains (H17, LM49), ^{c} bacteriophage isolated using an adherent-invasive *E. coli* (LF82) and active on VAP strain RY09. | | | | | |

### REFERENCES:

Throughout this application, various references describe the state of the art to which this invention pertains.
1. Tenaillon O, Skurnik D, Picard B, & Denamur E (2010) The population genetics of commensal Escherichia coli. Nat Rev Microbiol. 8(3):207-217. doi: 210.1038/nrmicro2298.
2. Nicolas-Chanoine MH, Bertrand X, & Madec JY (2014) Escherichia coli ST131, an Intriguing Clonal Group. Clin Microbiol Rev. 27(3):543-574.
3. Rogers BA, Sidjabat HE, & Paterson DL (2011) Escherichia coli O25b-ST131: a pandemic, multiresistant, community-associated strain. J Antimicrob Chemother. 66(1):1-14. doi: 10.1093/jac/dkq1415. Epub 2010 Nov 1016.
4. Russo TA & Johnson JR (2000) Proposal for a new inclusive designation for extraintestinal pathogenic isolates of Escherichia coli: ExPEC. J Infect Dis. 181(5):1753-1754. Epub 2000 May 1715.
5. Picard B, et al. (1999) The link between phylogeny and virulence in Escherichia coli extraintestinal infection. Infect Immun. 67(2):546-553.
6. Mora A, et al. (2014) Virulence patterns in a murine sepsis model of ST131 Escherichia coli clinical isolates belonging to serotypes O25b:H4 and O16:H5 are associated to specific virotypes. PLoS One. 9(1):e87025. doi: 87010.81371/journal.pone.0087025. eCollection 0082014.
7. Johnson JR, Porter SB, Zhanel G, Kuskowski MA, & Denamur E (2012) Virulence of Escherichia coli clinical isolates in a murine sepsis model in relation to sequence type ST131 status, fluoroquinolone resistance, and virulence genotype. Infect Immun. 80(4): 1554-1562. doi: 1510.1128/IAI.06388-06311. Epub 02012 Feb 06386.
8. Peirano G, et al. (2014) Global incidence of carbapenemase-producing Escherichia coli ST131. Emerg Infect Dis 20(11):1928-1931.
9. Coque TM, et al. (2008) Dissemination of clonally related Escherichia coli strains expressing extended-spectrum beta-lactamase CTX-M-15. Emerg Infect Dis. 14(2): 195-200. doi: 110.3201/eid1402.070350.
10. Nicolas-Chanoine MH, et al. (2008) Intercontinental emergence of Escherichia coli clone O25:H4-ST131 producing CTX-M-15. J Antimicrob Chemother. 61(2):273-281. Epub 2007 Dec 2011.
11. Colomer-Lluch M, et al. (2013) Detection of quinolone-resistant Escherichia coli isolates belonging to clonal groups O25b:H4-B2-ST131 and O25b:H4-D-ST69 in raw sewage and river water in Barcelona, Spain. J Antimicrob Chemother 68(4):758-765.
12. Reardon S (2014) Phage therapy gets revitalized. Nature 510(7503):15-16.
13. Lefort A, et al. (2011) Host factors and portal of entry outweigh bacterial determinants to predict the severity of Escherichia coli bacteremia. J Clin Microbiol. 49(3):777-783. doi: 710.1128/JCM.01902-01910. Epub 02010 Dec 01922.
14. Messika J, et al. (2012) Pathophysiology of Escherichia coli ventilator-associated pneumonia: implication of highly virulent extraintestinal pathogenic strains. Intensive Care Med 38(12):2007-2016.
15. Smati M, et al. (2013) Real-time PCR for quantitative analysis of human commensal Escherichia coli populations reveals a high frequency of subdominant phylogroups. Appl Environ Microbiol. 79(16):5005-5012. doi: 5010.1128/AEM.01423-01413. Epub 02013 Jun 01414.
16. Ochman H & Selander RK (1984) Standard reference strains of Escherichia coli from natural populations. J Bacteriol. 157(2):690-693.
17. Clermont O, Christenson JK, Denamur E, & Gordon DM (2013) The Clermont Escherichia coli phylo-typing method revisited: improvement of specificity and detection of new phylo-groups. Environ Microbiol Rep. 5(1):58-65. doi: 10.1111/1758-2229.12019. Epub 12012 Dec 12024.
18. Clermont O, Gordon D, & Denamur E (2015) A guide to the various phylogenetic classification schemes for Escherichia coli and the correspondence among schemes. Microbiology 161 (Pt 5):980-988.
19. Clermont O, Johnson JR, Menard M, & Denamur E (2007) Determination of Escherichia coli O types by allele-specific polymerase chain reaction: application to the O types involved in human septicemia. Diagn Microbiol Infect Dis. 57(2):129-136. Epub 2006 Oct 2003.
20. Clermont O, et al. (2011) Animal and human pathogenic Escherichia coli strains share common genetic backgrounds. Infect Genet Evol. 11(3):654-662. doi: 610.1016/j.meegid.2011.1002.1005. Epub 2011 Feb 1013.
21. Szijarto V, et al. (2014) Diagnostic potential of monoclonal antibodies specific to the unique O-antigen of multidrug-resistant epidemic Escherichia coli clone ST131-O25b:H4. Clin Vaccine Immunol. 21(7):930-939. doi: 910.1128/CVI.00685-00613. Epub 02014 Apr 00630.
22. Boulanger P (2009) Purification of bacteriophages and SDS-PAGE analysis of phage structural proteins from ghost particles. Methods Mol Biol 502:227-238.
23. Saussereau E, et al. (2014) Effectiveness of bacteriophages in the sputum of cystic fibrosis patients. Clin Microbiol Infect. 20(12):O983-990. doi: 910.1111/1469-0691.12712. Epub 12014 Jul 12726.
24. Whitfield C (2006) Biosynthesis and assembly of capsular polysaccharides in Escherichia coli. Annu Rev Biochem 75:39-68.
25. Davis MR, Jr. & Goldberg JB (2012) Purification and visualization of lipopolysaccharide from Gram-negative bacteria by hot aqueous-phenol extraction. J Vis Exp. (63).(pii):3916. doi: 3910.3791/3916.
26. Hyman P & Abedon ST (2009) Practical methods for determining phage growth parameters. Methods Mol Biol. 501:175-202.(doi):10.1007/1978-1001-60327-60164-60326_60318.
27. Pickard DJ (2009) Preparation of bacteriophage lysates and pure DNA. Methods Mol Biol. 502:3-9.(doi):10.1007/1978-1001-60327-60565-60321_60321.
28. Aziz RK, et al. (2008) The RAST Server: rapid annotations using subsystems technology. BMC Genomics 9:75.
29. Vallenet D, et al. (2013) MicroScope--an integrated microbial resource for the curation and comparative analysis of genomic and metabolic data. Nucleic Acids Res 41(Database issue):D636-647.
30. Dufour N, Debarbieux L, Fromentin M, & Ricard JD (2015) Treatment of Highly Virulent Extraintestinal Pathogenic Escherichia coli Pneumonia With Bacteriophages. Crit Care Med 43(6):e190-198.
31. Vimont S, et al. (2012) The CTX-M-15-producing Escherichia coli clone O25b: H4-ST131 has high intestine colonization and urinary tract infection abilities. PLoS One 7(9):e46547.
32. Johnson JR, et al. (2013) Abrupt emergence of a single dominant multidrug-resistant strain of Escherichia coli. J Infect Dis 207(6):919-928.
33. Scholl D & Merril C (2005) The genome of bacteriophage K1F, a T7-like phage that has acquired the ability to replicate on K1 strains of Escherichia coli. J Bacteriol 187(24):8499-8503.
34. Kajsik M, et al. (2014) Characterization and genome sequence of Dev2, a new T7-like bacteriophage infecting Cronobacter turicensis. Arch Virol 159(11):3013-3019.
35. Steven AC, et al. (1988) Molecular substructure of a viral receptor-recognition protein. The gp17 tail-fiber of bacteriophage T7. J Mol Biol 200(2):351-365.
36. Casjens SR & Molineux IJ (2012) Short noncontractile tail machines: adsorption and DNA delivery by podoviruses. Adv Exp Med Biol 726:143-179.
37. Kelley LA, Mezulis S, Yates CM, Wass MN, & Sternberg MJ (2015) The Phyre2 web portal for protein modeling, prediction and analysis. Nat Protoc 10(6):845-858.
38. Adams MH & Park BH (1956) An enzyme produced by a phage-host cell system. II. The properties of the polysaccharide depolymerase. Virology 2(6):719-736.
39. Szijarto V, et al. (2015) Bactericidal monoclonal antibodies specific to the lipopolysaccharide O antigen from multidrug-resistant Escherichia coli clone ST131-O25b:H4 elicit protection in mice. Antimicrob Agents Chemother 59(6):3109-3116.
40. Manges AR, et al. (2001) Widespread distribution of urinary tract infections caused by a multidrug-resistant Escherichia coli clonal group. N Engl J Med 345(14):1007-1013.
41. Riley LW (2014) Pandemic lineages of extraintestinal pathogenic Escherichia coli. Clin Microbiol Infect 20(5):380-390.
42. Johnson JR, et al. (2014) Rapid and specific detection, molecular epidemiology, and experimental virulence of the 016 subgroup within Escherichia coli sequence type 131. J Clin Microbiol 52(5):1358-1365.
43. Khan Mirzaei M & Nilsson AS (2015) Isolation of phages for phage therapy: a comparison of spot tests and efficiency of plating analyses for determination of host range and efficacy. PLoS One 10(3):e0118557.
44. Abedon ST (2011) Lysis from without. Bacteriophage. 1(1):46-49.
45. Heineman RH & Bull JJ (2007) Testing optimality with experimental evolution: lysis time in a bacteriophage. Evolution 61(7):1695-1709.
46. Nguyen HM & Kang C (2014) Lysis delay and burst shrinkage of coliphage T7 by deletion of terminator Tphi reversed by deletion of early genes. J Virol 88(4):2107-2115.
47. Bayer ME (1968) Adsorption of bacteriophages to adhesions between wall and membrane of Escherichia coli. J Virol 2(4):346-356.
48. Olkkonen VM & Bamford DH (1989) Quantitation of the adsorption and penetration stages of bacteriophage phi 6 infection. Virology 171(1):229-238.
49. Puck TT, Garen A, & Cline J (1951) The mechanism of virus attachment to host cells. I. The role of ions in the primary reaction. J Exp Med 93(1):65-88.
50. Storms ZJ, Smith L, Sauvageau D, & Cooper DG (2012) Modeling bacteriophage attachment using adsorption efficiency. Biochemical Engineering Journal 64:22-29.
51. De Paepe M & Taddei F (2006) Viruses' life history: towards a mechanistic basis of a trade-off between survival and reproduction among phages. PLoS Biol 4(7):e193.
52. Labrie SJ, Samson JE, & Moineau S (2010) Bacteriophage resistance mechanisms. Nat Rev Microbiol 8(5):317-327.
53. Jann K & Jann B (1987) Polysaccharide antigens of Escherichia coli. Rev Infect Dis 9 Suppl5:S517-526.
54. Bull JJ, Vimr ER, & Molineux IJ (2010) A tale of tails: Sialidase is key to success in a model of phage therapy against K1-capsulated Escherichia coli. Virology 398(1):79-86.
55. Hughes KA, Sutherland IW, Clark J, & Jones MV (1998) Bacteriophage and associated polysaccharide depolymerases--novel tools for study of bacterial biofilms. J Appl Microbiol 85(3):583-590.
56. Lin TL, et al. (2014) Isolation of a bacteriophage and its depolymerase specific for K1 capsule of Klebsiella pneumoniae: implication in typing and treatment. J Infect Dis 210(11):1734-1744.
57. Mushtaq N, Redpath MB, Luzio JP, & Taylor PW (2005) Treatment of experimental Escherichia coli infection with recombinant bacteriophage-derived capsule depolymerase. J Antimicrob Chemother 56(1):160-165.
58. Born Y, et al. (2014) The tail-associated depolymerase of Erwinia amylovora phage L1 mediates host cell adsorption and enzymatic capsule removal, which can enhance infection by other phage. Environ Microbiol 16(7):2168-2180.
59. Schmerer M, Molineux IJ, & Bull JJ (2014) Synergy as a rationale for phage therapy using phage cocktails. PeerJ 2:e590.
60. Henry M, Lavigne R, & Debarbieux L (2013) Predicting in vivo efficacy to guide the choice of therapeutic bacteriophages to treat pulmonary infections. Antimicrob Agents Chemother.
61. Cairns BJ, Timms AR, Jansen VA, Connerton IF, & Payne RJ (2009) Quantitative models of in vitro bacteriophage-host dynamics and their application to phage therapy. PLoS Pathog 5(1):e1000253.
62. Payne RJ & Jansen VA (2001) Understanding bacteriophage therapy as a density-dependent kinetic process. J Theor Biol 208(1):37-48.
63. Weld RJ, Butts C, & Heinemann JA (2004) Models of phage growth and their applicability to phage therapy. J Theor Biol 227(1):1-11.
64. Maura D, et al. (2012) Intestinal colonization by enteroaggregative Escherichia coli supports long-term bacteriophage replication in mice. Environ Microbiol. 14(8):1844-1854. doi: 1810.1111/j.1462-2920.2011.02644.x. Epub 02011 Nov 02628.
65. European Medicines Agency (2015) Workshop on the therapeutic use of bacteriophages.
66. Scholl D, Gebhart D, Williams SR, Bates A, & Mandrell R (2012) Genome sequence of E. coli O104:H4 leads to rapid development of a targeted antimicrobial agent against this emerging pathogen. PLoS One 7(3):e33637.
67. Yosef I, Manor M, Kiro R, & Qimron U (2015) Temperate and lytic bacteriophages programmed to sensitize and kill antibiotic-resistant bacteria. Proc Natl Acad Sci U S A 112(23):7267-7272.
68. Lu TK & Collins JJ (2007) Dispersing biofilms with engineered enzymatic bacteriophage. Proc Natl Acad Sci U S A 104(27):11197-11202.
69. Ando H, Lemire S, Pires DP, & Lu TK (2015) Engineering Modular Viral Scaffolds for Targeted Bacterial Population Editing. Cell Systems 1(3):187-196.
70. Joensen KG, et al. (2014) Real-time whole-genome sequencing for routine typing, surveillance, and outbreak detection of verotoxigenic Escherichia coli. J Clin Microbiol 52(5):1501-1510.
71. Zankari E, et al. (2012) Identification of acquired antimicrobial resistance genes. J Antimicrob Chemother 67(11):2640-2644.

## Claims

1. A polypeptide comprising an amino acid sequence having at least 80% of identity with the amino acid sequence set forth in SEQ ID NO: 1.

2. The polypeptide of claim 1 which is fused to at least one heterologous polypeptide.

3. The polypeptide of claim 1 which is fused to an immunoglobulin domain such as a Fc portion.

4. The polypeptide of claim 1 which is fused to a fluorescent polypeptide or to an enzyme.

5. A nucleic acid molecule which encodes for the polypeptide of claim 1.

6. The nucleic acid molecule of claim 5 which is included in a suitable vector, such as a plasmid, cosmid, episome, artificial chromosome, phage or a viral vector.

7. The polypeptide of claim 1 which is conjugated with a detectable label.

8. The polypeptide of claim 7 wherein the label is a fluorescent label.

9. The polypeptide of claim 1 which is biotinylated.

10. The polypeptide of claim 1 which is conjugated to a latex particle, a metal colloid particle, or a carbon nanotube.

11. A method for detecting the presence of the E coli 025b-ST131 clone in a sample comprising i) contacting the sample with the polypeptide as defined in any one claims 1 to 10 which is capable of forming complexes with the lipopolysaccharide (LPS) molecules of the E coli 025b-ST131 clone and ii) detecting the presence of the one or more said complexes wherein the presence of at least one complex indicates the presence of the clone in the sample.

12. The method of claim 11 wherein the sample is urine, blood, serum, blood products, plasma, saliva, body fluid, water, culture medium, diluted culture medium, petroleum product, fuel, liquid undergoing fermentation, or a beverage.

13. The method of claim 11 wherein the sample is human or animal tissue, stool, sputum, expectorate, an agricultural product, food, solids collected by centrifugation or filtration, soil, or sediment.

14. A lateral flow device comprising the polypeptide of claim 1.

## Patentansprüche

1. Polypeptid, das eine Aminosäuresequenz umfasst, die mindestens 80 % Identität mit der in SEQ ID Nr. 1 dargelegten Aminosäuresequenz aufweist.

2. Polypeptid nach Anspruch 1, das an mindestens ein heterologes Polypeptid fusioniert ist.

3. Polypeptid nach Anspruch 1, das an eine Immunglobulindomäne, wie etwa einen Fc-Teil, fusioniert ist.

4. Polypeptid nach Anspruch 1, das an ein Fluoreszenz-Polypeptid oder an ein Enzym fusioniert ist.

5. Nukleinsäuremolekül, das für das Polypeptid nach Anspruch 1 codiert.

6. Nukleinsäuremolekül nach Anspruch 5, das in einem geeigneten Vektor, wie etwa einem Plasmid, Cosmid, Episom, künstlichen Chromosom, Phagen oder einem viralen Vektor, eingeschlossen ist.

7. Polypeptid nach Anspruch 1, das mit einer nachweisbaren Markierung konjugiert ist.

8. Polypeptid nach Anspruch 7, wobei es sich bei der Markierung um eine Fluoreszenzmarkierung handelt.

9. Polypeptid nach Anspruch 1, das biotinyliert ist.

10. Polypeptid nach Anspruch 1, das an ein Latexpartikel, ein Metallkolloidpartikel oder ein Kohlenstoffnanoröhrchen konjugiert ist.

11. Verfahren zum Nachweis des Vorhandenseins des E. coli O25b-ST131-Klons in einer Probe, umfassend i) das Inkontaktbringen der Probe mit dem Polypeptid wie in einem der Ansprüche 1 bis 10 definiert, das in der Lage ist, Komplexe mit den Lipopolysaccharid- (LPS) Molekülen des E. coli O25b-ST131-Klons zu bilden, und ii) das Nachweisen des Vorhandenseins des einen oder mehrerer der Komplexe, wobei das Vorhandensein mindestens eines Komplexes das Vorhandensein des Klons in der Probe anzeigt.

12. Verfahren nach Anspruch 11, wobei es sich bei der Probe um Urin, Blut, Serum, Blutprodukte, Plasma, Speichel, Körperfluid, Wasser, Kulturmedium, verdünntes Kulturmedium, Erdölprodukt, Kraftstoff, Fermentation durchlaufende Flüssigkeit oder ein Getränk handelt.

13. Verfahren nach Anspruch 11, wobei es sich bei der Probe um menschliches oder tierisches Gewebe, Stuhl, Sputum, Expektorat, ein landwirtschaftliches Produkt, Lebensmittel, durch Zentrifugation oder Filtration abgesammelte Feststoffe, Erde oder Sediment handelt.

14. Lateralflussvorrichtung, die das Polypeptid nach Anspruch 1 umfasst.

## Revendications

1. Polypeptide comprenant une séquence d'acides aminés présentant au moins 80 % d'identité avec la séquence d'acides aminés décrite dans SEQ ID NO : 1.

2. Polypeptide selon la revendication 1 qui est fusionné à au moins un polypeptide hétérologue.

3. Polypeptide selon la revendication 1 qui est fusionné à un domaine d'immunoglobuline tel qu'une portion Fc.

4. Polypeptide selon la revendication 1 qui est fusionné à un polypeptide fluorescent ou à une enzyme.

5. Molécule d'acide nucléique qui code pour le polypeptide selon la revendication 1.

6. Molécule d'acide nucléique selon la revendication 5 qui est inclus dans un vecteur adapté, tel qu'un plasmide, un cosmide, un épisome, un chromosome artificiel, un phage ou un vecteur viral.

7. Polypeptide selon la revendication 1 qui est conjugué à un marqueur détectable.

8. Polypeptide selon la revendication 7 dans lequel le marqueur est un marqueur fluorescent.

9. Polypeptide selon la revendication 1 qui est biotinylé.

10. Polypeptide selon la revendication 1 qui est conjugué à une particule de latex, une particule de colloïde de métal, ou un nanotube de carbone.

11. Procédé de détection de la présence du clone O25b-ST131 d'E coli dans un échantillon comprenant i) la mise en contact de l'échantillon avec le polypeptide selon l'une quelconque des revendications 1 à 10 qui est capable de former des complexes avec les molécules de lipopolysaccharide (LPS) du clone O25b-ST131 d'E coli et ii) la détection de la présence de l'un ou plusieurs desdits complexes dans lequel la présence d'au moins un complexe indique la présence du clone dans l'échantillon.

12. Procédé selon la revendication 11 dans lequel l'échantillon est de l'urine, du sang, du sérum, des produits sanguins, du plasma, de la salive, un fluide corporel, de l'eau, un milieu de culture, un milieu de culture dilué, un produit pétrolier, un combustible, un liquide subissant une fermentation, ou une boisson.

13. Procédé selon la revendication 11 dans lequel l'échantillon est un tissu humain ou animal, des selles, un crachat, un expectorat, un produit agricole, un aliment, des solides collectés par centrifugation ou filtration, du sol, ou un sédiment.

14. Dispositif de flux latéral comprenant le polypeptide selon la revendication 1.
